(19)

Europäisches
Patentamt
European
Patent Office
Office européen
des brevets

(11)  **EP 2 868 752 A1**

(12)  **EUROPEAN PATENT APPLICATION**

(43) Date of publication:
06.05.2015  Bulletin 2015/19

(51) Int Cl.:
*C12Q 1/68* (2006.01)  *G06F 19/22* (2011.01)

(21) Application number: 13191170.3

(22) Date of filing: 31.10.2013

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Lexogen GmbH
1030 Vienna (AT)**

(72) Inventor: **Türk, Andreas
1020 Wien (AT)**

(74) Representative: **Sonn & Partner Patentanwälte
Riemergasse 14
A-1010 Wien (AT)**

(54)  **Nucleic acid copy number determination based on fragment estimates**

(57)  The present invention provides a method for determining an amount of nucleic acid molecule copies of a preselected nucleic acid molecule in a sample in a fragmentation based method, comprising the steps of providing the fragment amount for said preselected nucleic acid molecule, a distribution of fragment coverage over a genetic coordinate for said preselected nucleic acid molecule, obtaining a plurality of virtual fragments, assembling the obtained virtual fragments, obtaining the amount of assembled virtual fragments per assembled sequence, whereby the amount of the copies of a preselected nucleic acid molecule in a sample can be calculated from the fragment quantitiy and the amount of assembled virtual fragments per assembled sequence.

EP 2 868 752 A1

**Description**

**Field of the invention**

The present invention relates to providing information of transcript (e.g. mRNA) copy numbers based on fragmentation based sequencing methods, such as next generation sequencing (NGS).

**Background**

Next generation sequencing technology produces a large amount of short reads when sequencing a nucleic acid sample. An essential step in next generation sequencing is the library preparation or library prep for short. This process takes mRNA or cDNA as input and produces a library of short cDNA fragments, each corresponding to a section of an mRNA molecule. These fragments are then sequenced by an NGS sequencer, usually not in their entirety but partially at their start and/or at their end. This results in short sequences of nucleotides which are called reads and are most commonly stored by the NGS sequencer as sequences of a group of four ASCII characters such as A, C, G, T or 0, 1, 2, 3, representing the nucleobases of the genetic code. In order to infer which mRNA molecules were present in the original sample, the reads are mapped onto a reference genome.

Next generation sequencing has been employed in a variety of genome mapping procedures (US 2013/110410 A1) or DNA identification methods, e.g. by using a mapped genome to associate sequence reads to a certain organism variant (WO 2009/085412 A1).

WO 2009/091798 A1 describes a method for obtaining a profile of a transcriptome of an organism, the method comprising: sequencing one or more cDNA molecules to obtain sequencing reads; aligning each of the sequencing reads to a reference sequence.

The estimate of the number of copies of a transcript based on the fragment sequence synthesis (FSS) is a within-sample normalization method since it normalizes the read counts of different transcripts from a single sample. Within-sample normalization is one aspect of the FPKM [5] and length adjusted FPKM [9] values, which they achieve through dividing the number of reads assigned to a transcript by the transcript length and adjusted transcript length. Both FPKM and length adjusted FPKM values cannot correct for variations in the distribution of fragments between different transcripts. Furthermore, FPKM and adjusted length FPKM values are no estimates of the number of copies of a transcript but are abstract measures correlated to the latter. Other normalization methods in the literature such as [1, 2, 8] are between sample normalization methods which account for the technical and/or biological variability in the number of reads generated by a transcript or gene.

Despite improvements in read assembly, methods to determine transcript copy quantities based on fragmentation data are still inaccurate and further improvements are needed. It is a goal of the present invention to provide improved methods that allow a more accurate assessment of transcript abundances.

**Summary of the invention**

The present invention provides a method for determining an amount of nucleic acid molecule copies of a preselected nucleic acid molecule in a sample, e.g. in a fragmentation based method, comprising the steps of

a) fragmenting the preselected nucleic acid molecule or generating fragments of the preselected nucleic acid molecule,
b) providing the amount (e.g. quantity or also concentration) of fragments for said preselected nucleic acid molecule,
c) providing a distribution of fragment coverage over a genetic coordinate for said preselected nucleic acid molecule,
d1) generating a plurality of virtual fragments based on the genetic coordinates of the preselected nucleic acid molecule, so that the generated virtual fragments have substantially the distribution of fragment coverage over the genetic coordinate according to step c), or d2) determining the nucleic acid sequence of fragments obtained in step a) and providing each nucleic acid sequence of a fragment as virtual fragment;
thereby obtaining a plurality of virtual fragments,
e) assembling the obtained virtual fragments from step d) into fragment sequences, wherein each virtual fragment is assembled into a fragment sequence which is composed of matching virtual fragments, until all virtual fragments of step d) have been either assembled into a fragment sequence and/or do not match with any other non-assembled virtual fragment or fragment sequence and all fragment sequences do not match with any other fragment sequence, wherein each final fragment sequence and non-assembled virtual fragment is regarded as a virtual copy,
f) obtaining the amount, preferably an average or a distribution or the quantity, of virtual fragments from step d) per virtual copy obtained in step e),
g) whereby the amount, preferably an average or a distribution or quantity, of the copies of the preselected nucleic acid molecule in a sample can be calculated from the fragment amount of step b) and the amount of fragments per virtual copy of step f).

The invention further provides a computer program product employing said method, e.g. containing machine code to perform or assist in said methods and steps on a computer. The computer program product can be provided on any kind of memory device. Also provided is a system, e.g. a computer device, programmed to assist in performing the steps of the inventive method. Calculation steps are usually performed without assistance of an operator. Input and setting steps may be assisted by the program or system, e.g. by suggesting an option proposal for the number random step repetitions, if desired. Of course, the program or system may also be performed with default parameters without further input from an operator.

The following detailed description and preferred embodiments apply to all aspects of the invention and can be combined with each other without restriction, except were explicitly indicated. Preferred embodiments and aspects are defined in the claims.

## Detailed disclosure of the invention

The present invention is based on a new method that allows estimation of nucleic acid molecule copy amounts in a sample. The nucleic acid molecule copies are substantially identical nucleic acid molecules, having the same nucleic acid sequence. E.g., the nucleic acid molecule can be selected from a DNA, e.g. cDNA, or RNA molecule, preferably a transcript, especially an mRNA. In this case, the present application also refers to transcript copy numbers, however, the concept is described for transcript copies but also applies for any other nucleic acid molecule and such disclosure shall not be considered to be limited to analysing transcript copies. The amount, e.g. a quantity, of the preselected nucleic acid molecule may also be referred to herein as "copy number".

The invention can determine the amount or concentration of a given nucleic acid molecule and its copies in a sample. A sample may comprise also other nucleic acid molecules with other sequences. However, the present invention is suitable to distinguish between such nucleic acids and the nucleic acid molecule to be the subject of the present method, which is hence referred to as "preselected" nucleic acid molecule. Usually the preselected nucleic acid molecule has a known nucleic acid sequence, e.g. known from deposited database sequence data, e.g. at the NCBI or EBI.

Any nucleic acid molecule can be used with any length, which may be fragmented. Preferably, the preselected nucleic acid molecule has 500 to 10000000 nucleotides in length, preferably 1000 to 1000000 nucleotides in length, even more preferred 5000 to 500000 nucleotides in length.

The present invention is based on the analysis of nucleic acid fragment data and is usually used in the context of a fragmentation based sequencing method, such as next generation sequencing. It is possible to supplement such methods with the inventive method to determine nucleic acid copy numbers (i.e. amounts). It is noteworthy, that the present invention is not dependent on fragment sequence data and can work with or without sequence information of the fragments. E.g. the virtual fragments may not be defined by a nucleic acid sequence but simply by the indication of the start and end position on the genetic coordinate (or alternatively, the start or end position and the length in nucleotides, which is equivalent). Essentially, during the inventive method steps d) to g), the nucleic acid sequence information of the virtual fragments is not needed anymore and may not be used during the calculation in any one of these steps, especially in step d1) and/or e). Of course, alternatively, if the nucleic acid sequence of a fragment is known, by using the reference sequence of the preselected nucleic acid of interest it is also possible to calculate the position information for such a fragment and vice-versa to determine the nucleic acid sequence of any virtual fragment for which the position data is known. Matching nucleotides according to step e) can be simply determined with the position information (i.e. start and end position) of the virtual fragments.

Instead (or in addition), the present invention uses the fragmentation profile of the (preselected) nucleic acid molecule when determining the copy number. To this goal, in most embodiments the present invention uses statistical methods, which may result in a probability for copy numbers, which may be a likelihood distribution of copy numbers.

Fragments can be generated by any known means. Preferred is a random fragmentation, such as a sequence non-specific fragmentation, although some bias for particular nucleotide sections with preferred (but not exclusive) cutting sites may exist, such as nucleic acid molecule portions with reduced stability that are easily cut. Random fragmentation is to be distinguished from specific fragmentation, e.g. by using restriction enzymes, which cut exclusively at defined restriction sites. Preferably the fragmentation is by physical means, in particular preferred by sonication, shearing or elevated temperature. Another option to generate fragments is to amplify partial copies with partial sequences of the preselected nucleic acid molecule. Random fragments can be generated in this way by e.g. using random primers, such as random hexamers. Sequence dependent fragmenting can be controlled by selecting template portion specific primers. In another embodiment only partial sequences are generated during sequencing, e.g. by random priming during sequencing. This approach will simultaneously result in fragment nucleic acid sequences provided in step d2). Thus partial sequences of fragments can be generated, which is sufficient for the methods of the present invention. Amplification of fragments and fragment sequencing may result in fragments in other concentrations as would have occurred by fragmenting the preselected nucleic acid molecule itself, however any biases created in this way are conveniently cancelled during the inventive method, e.g. in step e) since the assembly of fragments into fragment sequences used in this step automatically results in a reduction of the bias.

The fragments of step a) and/or the virtual fragments of step d) may have an average size of 40 to 2000 nucleotides in length, preferably 60 to 1000 nucleotides in length, especially preferred 80 to 800 nucleotides in length. The fragment sizes for step a) (fragmenting) and d) (a step in the analysis) may be selected dependent on the respective other step or independently. During step a) fragmentation sizes may be controlled by e.g. the number of different restriction enzymes, the temperature, sonification volume, shear stress and /or the length of treatment, especially in case of physical means.

According to step b) the amount of fragments is determined. The amount can be represented by an absolute count of fragments, a concentration or a molar quantity, in absolutes or relative to other (e.g. the total) nucleic acids in the sample. The reference to a volume or other nucleic acids is essentially non-consequential when considered in further calculations. According to the invention it is usually most convenient to operate with a fragment quantity or a fraction thereof, which then of course needs to be considered and multiplied in the final copy number determination.

The fragmentation profile is an estimate for the distribution of the fragment location over the length of the preselected nucleic acid molecule. Locations over the length of a nucleic acid molecule are represented as genetic coordinates, which for mRNA transcripts extend from 1 to the number of the last (3') nucleotide. The fragmentation profile is then a distribution of fragment coverage over a genetic coordinate for said preselected nucleic acid molecule. This distribution or profile is not required to be an exact representation of each and every fragment of the preselected nucleic acid molecule that provides an estimate of differences in the fragment coverage over the genetic coordinate. It is possible that fragment coverage is uniform over the entire length of the nucleic acid molecule, but it is also possible that particular portions of the nucleic acid molecule are over- or underrepresented by fragments, e.g. in certain profiles the 3' end and/or 5' end are over-represented in comparison to the middle portion. If not considered, such different profiles may distort a correct estimate of the copy numbers. Fragment coverage may be represented by a coverage envelope curve (also referred to as total coverage histogram or histogram of total) to the individual fragment coverage values.

To calculate the fragment coverage it is not required to consider each and every nucleotide for each and every fragment. It is sufficient to consider e.g. fragment start site distributions or a distribution of at least one nucleotide of each fragment at a preselected position from a fragment 3' or 5' terminus on the genetic coordinate. The "at least one nucleotide" can e.g. be the 3' terminal nucleotide (start site) or any other nucleotide, e.g. the nucleotide at position 1, 2, 3, 4, 5, 6 , 7, 8, 9 or 10 or any combination thereof from either the 3' end and/or 5' end of the fragment. The distribution may of course include all nucleotides of the fragments. This (these) nucleotide(s) should be the same position(s) for all fragments under consideration.

Several methods exist to determine the distribution of fragment coverage over a genetic coordinate. One method is disclosed in European patent application EP 13175774.2 (incorporated herein by reference). The method may comprise assigning fragment sequencing data to genetic coordinates of a preselected nucleic acid molecule thereby obtaining a data set of fragment genetic coordinate coverage. A fragment distribution can be modeled by e.g. pre-setting a probability distribution function of modeled genetic coverage for a nucleic acid molecule, wherein said probability distribution function is defined by a weight factor $\alpha i$ of said nucleic acid molecule multiplied with the sum of at least 2 (e.g. 2, 3, 4, 5, 6, 7, 8 or more) probability subfunctions j, with j denoting the numerical identifier for a probability subfunction, each probability subfunction j being independently weighted by a weight factor $\beta j$. The probability subfunctions can e.g. be simple functions like an aperiodic function, preferably a Gaussian function, a square shape function, a triangle shape function, especially preferred a Gaussian function. In general, the probability subfunction is constituted of positive values for each genetic coordinate. Preferably it is a density function. The sum of such modeled subfunctions can be the distribution of fragment coverage as used according to the inventive method.

As is apparent in view of the suitability of such modeled functions, the inventive fragment coverage may also be an approximation or local average, deviating from the exact count of fragment coverage. Such an approach may e.g. be an average number over 2 to 600, preferably 10 to 500, more preferred 20 to 400, even more preferred 30 to 300, or 40 to 200, or 50 to 100, adjacent nucleic acid nucleotides of the preselected nucleic acid molecule.

It is also possible to determine the distribution of fragment coverage over a genetic coordinate by using a reference nucleic acid. The reference nucleic acid is fragmented, the coverage of fragments over a genetic coordinate of said reference nucleic acid is determined, thereby obtaining a reference coverage. If the reference molecule differs in length to the preselected nucleic acid molecule, it is possible to fit, e.g. stretch or shrink, the reference coverage to the genetic coordinate of the preselected nucleic acid molecule. Reference nucleic acid molecules can e.g. be a reference, which fragments do not align with human or mammalian nucleic acid molecules. Such non-aligning reference nucleic acid molecules have been made available by the ERCC (Qu et al., Journal of Biomolecular Techniques 2011, 22, supplement, S46) and are provided by Lifetechnologies as ERCC spikes (Lifetechnologies, USA, Catalog no. 4456740, 4456739). The reference nucleic acid should resemble the fragmentation behavior of the preselected nucleic acid molecule and have e.g. similar length, such as a length with less than 10 times, less than 8 times, less than 6 times, less than 4 times or less than 2 times differences in length. Preferably the reference nucleic acid has a similar GC-content as the preselected nucleic acid molecule, such as a GC content which differs by less than 20%, less than 50%, less than 10%, less than 8%, less than 6% in the GC-content. The reference nucleic acid may be present in the same sample, as the preselected nucleic acid molecule, or it may be in a different sample. If in a different sample, the different sample is preferably handled similarly as the sample with the preselected nucleic acid molecule, e.g. by using the same experimental protocol, to ensure a similar fragmentation condition. The reference nucleic acid molecule may be labeled so as to distinguish it from other nucleic acids in the sample. Such labels may include radio labels or fluorescence labels or preferably nucleic acid barcodes, which may be transferred to the fragments as disclosed in WO 2011/070155.

A further possibility to provide a distribution of fragment coverage over the genetic coordinate for the preselected nucleic acid molecule comprises isolating the preselected nucleic acid molecule from the sample, generating fragments and analysing the fragment distribution in view of the sequence of the preselected nucleic acid molecule. Instead of isolating the preselected nucleic acid molecule it is also possible to label the preselected nucleic acid molecule so that the fragments can be distinguished and analysed separately from other nucleic acids or fragments potentially present in the sample.

With the distribution of fragment coverage determined for the preselected nucleic acid molecule as described above, it is possible to artificially generate virtual fragments of said preselected nucleic acid molecule. This is a conceptual step (which is indicated by "virtual") that is best performed on a computer. A virtual fragment has the information to define a fragment of the preselected nucleic acid molecule. This information can be simply a start and end position of the fragment on the genetic coordinate of said preselected nucleic acid molecule. It is possible, but not necessary to use the nucleic acid sequence of such as fragment. With the knowledge of the nucleic acid sequence of the preselected nucleic acid molecule it is possible to obtain the nucleic acid sequence from the position data and vice versa. The virtual fragments shall have a fragment distribution resembling the distribution of fragment coverage of the preselected nucleic acid molecule. I.e. the distribution of fragment coverage of the virtual fragments of step d1) and of the fragments of step c) is substantially the same. The distribution of fragment coverage of the virtual fragments can be generated by selecting suitable fragments, and controlled by again generating such a distribution by the same methods as described above for the fragments of the preselected nucleic acid molecule. The generation of virtual fragments is essentially a random process. At a random position in the genomic coordinates of the preselected nucleic acid molecule a fragment with random length is extracted. The length of the virtual fragments preferably resembles the length distribution of fragments obtained by the used fragmenting method employed in step a). Random virtual fragment generation does not mean that the parameter of a fragment are purely by chance, but usually the random generation of position parameters (such as start position and length or end position) may be weighted by the fragment distribution obtained in step c), e.g. a start site (or end site) and length distribution. Preferably the amount of artificially generated virtual fragments is sufficient so that generation of any additional virtual fragments has a small or negligible effect on the amount of assembled fragments obtained in step f), e.g. when the calculated result converges.

In preferred embodiments the inventive method further comprises a step of providing a distribution of fragment lengths of the fragments of step a) and wherein in step d1) the fragments are generated so that the plurality of generated virtual fragment sequences has substantially the distribution of fragment lengths. A distribution of fragment lengths can be determined by the same methods as described above for providing the distribution of fragment coverage or by studying a bioanalyser trace. It may result in a probability distribution function for fragment lengths.

"Substantially" and "about" as used herein may refer to the same value or a value differing by +/- 10% of the given value, or in the case of distribution functions, identical functions or functions with +/- 10% on average per coordinate, preferably also at most +/- 10% per coordinate.

In preferred embodiments of the invention it is possible to repeat this step d1) of generating a plurality of virtual fragments at least once, thereby obtaining a different set of generated virtual fragments for each repetition of step d1). Repetitions of the generation of virtual fragments leads to different sets of pluralities of artificial fragments, and allows a better statistical analysis of fragment representation, especially during fragment assembly in the next step e). Further repetitions can be performed in a statistically relevant and reliable amount. in preferred embodiments at least 20, preferably at least 50, even more preferred at least 200, random sets of generated virtual fragment sequences are generated.

In addition or alternatively, it is possible to continuously generate virtual fragments according to step d1) and assemble them according to step e), preferably until the amount of virtual fragments per virtual copy of step f) converges. It shall be noted that steps d) and e) can be perfomed repeatedly for each additional virtual fragment generated in step d), e.g. d) and e) may be performed cyclically. Preferably at least 10, especially preferred at least 20, at least 30, at least 40, at least 50, at least 60, at least 60, at least 80, at least 100, at least 125, at least 150, at least 200, at least 300, at least 400, or at least 500, virtual fragments per nucleotide in length of the preselected nucleic acid molecule are generated. The amount, in particular the mean value, of virtual fragments per virtual copy converges, when said amount remains substantially the same, preferably does not change by more than 5%, preferably not more than 3%, even more preferred not more than 2% in particular preferred not more than 1%.

In case the sequence information of the fragment is known e.g. after sequencing the fragments or via obtaining sequence information of the virtual fragments, it is also possible to simply use these sequences in the next step e) - but preferred is the use of position data of the virtual fragments. As is common for fragment sequencing data, it is possible that sequence information is only available for a fraction of the generated fragments. E.g. it is possible that in step d2) 1% to 100 %, preferably 2% to 80%, even more preferred 3% to 40% of the fragments are sequenced. It is also possible that only less than 50%, less than 40%, less than 30%, less than 20%, or less than 10%, of the fragments are sequenced.

The next step in the inventive method is the assembly of the artificial (d1) or non-artificial (d2) virtual fragments of any step d). This leads to fragment sequences, i.e. multiple series of virtual fragments, where a fragment sequence consists of at least one virtual fragment. Each virtual fragment within a virtual fragment can have an associated index indicating its position within the fragment sequence, i.e. a series of virtual fragments. Thus a "fragment sequence" of this (also conceptual step) is a series or a succession of fragments - not to be confused with nucleic acid sequences of fragments, which may not be used at all in the conceptual steps.

In the assembly step a fragment can be inserted at a specified position into a fragment sequence if the fragments start and end match or the resulting fragment sequence satisfies the assembly condition. This condition might refer to the relative position of the genome coordinates of the fragments within the fragment sequence or to the similarity of the nucleotide sequences of the fragments within the sequence. The nucleotide sequences of the fragments are available for fragments obtained in a) and sequenced in d2) but might not be available for artificially generated virtual fragments in d1). In preferred embodiments, the assembly condition is that two virtual fragments match as adjacent within one fragment sequence when the start in genome coordinates (or position) of a subsequent fragment follows immediately after the end in genome coordinates (or position) of a preceeding virtual fragment (exact match) or the start of a subsequent virtual fragment and the end of a preceeding virtual fragment are located within a window of +/- 1 to +/- 200 nucleotides (inexact match). This "window" can be seen as an overlap or gap imprecision, that surprisingly can provided faster and/or better results within the inventive method. This window is preferably a gap or overlap of the start (subsequent virtual fragment) to end (of the subsequent virtual fragment) positions of 1 to 150 nucleotides, e.g. 1, 2, 3, 4, 5, 6, 7, 8, 9, 10, 11, 12, 13, 14, 15, 16, 17, 18, 19, 20, e.g. up to 120, up to 100, up to 80, up to 60, up to 40, up to 30 or up to 25 nucleotides. Another condition for an inexact match is an overlap or gap imprecision of at most 10 % of the average virtual fragment length in nucleotides. In fact, allowing an inexact match improves performance of the inventive method, without substantially altering the outcome in step f) and g) of an assembly condition with exact match. If the nucleotide sequence of the fragments is known the assembly condition might require that parts of the nucleotide sequence of two fragments within one sequence adjacent in terms of their sequence indices are similar or identical potentially after reversion or reverse complementation of the nucleotide sequences. These or other conditions may be combined in the assembly condition. If the nucleotide sequence of the fragments is known their location in genome coordinates can be inferred by mapping their nucleotide sequence to a reference genome. This allows to determine whether assembly conditions referring to the relative location of the fragments within the fragment sequence are met.

The assembly step may be random. Two types of randomness can be included, the random generation of subsequently processed virtual fragments (or alternatively, if a plurality of fragments is generated at once and later assembled, the order of virtual fragments to be assembled can be random) and/or for a given virtual fragment the insertion into a fragment sequence randomly selected from the set of all fragment sequences previously generated by the assembly step, whose extension by the fragment meets the assembly condition.

The assembly step is repeated for all randomly or non-randomly generated virtual fragments until all fragments have been assembled or fail to match any fragment sequence or other non-assembled virtual fragments. Each fragment sequence or non-assembled virtual fragment in the final set of fragment sequences represents a virtual copy. For the inventive method, only the number of fragments per virtual copy, i.e. the number of fragments assembled to form a virtual copy is required. This number is calculated for each resulting virtual copy, which provides step f) the amount, e.g. an average or a distribution or the quantity, of assembled fragments from step d) per virtual copy obtained in step e).

In a particular embodiment in case of random factors in step e), preferably the assembly step e) is repeated with a different order of fragments preferably wherein at least 10 different fragments, or at least 100, especially at least 1000, different (e.g. random) fragments sequence sets are generated. Such different results may be further considered when calculating the average or distribution of fragments per virtual copy.

The amount of copies determined in step g) is a simple comparison of the amount of fragments as determined in step b) and the result of the calculated virtual fragments per virtual copy as determined in step f) - in view of step e). The amount of copies may be an absolute value or a relative value, dependent on the removal of any calculation bias and in dependence on the method used to generate fragments and the selected amount of virtual fragments as mentioned above. Given the statistical nature of the inventive method, the amount may be given as an average or a probability distribution of the quantity or concentration. A simple calculation to estimate the result is by providing the ratio of the fragment amount of step b) to the amount (or average) of annealed fragments per virtual copy of step f). It is also possible to estimate the probability of the copies of a preselected nucleic acid molecule from a distribution in step f) and the fragment amount of step b), e.g. using a posterior probability estimation. Any such method to transform the fragment amount with the amount of annealed fragments per virtual copy, wherein the latter could be a probability function itself, resulting in a probability function for the determined copy amount of the preselected nucleic acid molecule is possible.

The invention further relates to a computer readable memory device comprising a computer program product for performing a method according to the invention on a computer or for supporting a method according to the invention by a computer, especially any one of steps b), c), d1), d2), e), f) and g) may be assisted with a computer. Especially steps c), d1), e), f) and g) may be performed on a computer. Even the usually wet-chemistry step a) and/or b) of fragmenting the preselected nucleic acid molecule or step d2) of determining the nucleic acid sequence may be assisted by a computer, e.g. to control and obtain data from an automated or semi-automated sequence reader. The computer program product or memory device may also be provided with a sequence generation component that obtains short sequencing reads from a sample, such as a sequencer, preferably a sequencer comprising a computer component. For example, computer readable media can include but are not limited to magnetic storage devices (e.g., hard disk, floppy disk, magnetic strips, ...), optical disks (e.g., compact disk (CD), digital versatile disk (DVD), ...), smart cards, and flash memory devices (e.g., card, stick, key drive, ...).

The present invention is further illustrated by the following figures and examples, without being limited to these embodiments of the invention, with - each element being combinable with any other embodiment of the invention. Especially any one of the formulas provided below can be used separately, individually or in combination to describe or define a step in the inventive method.

**Figures:**

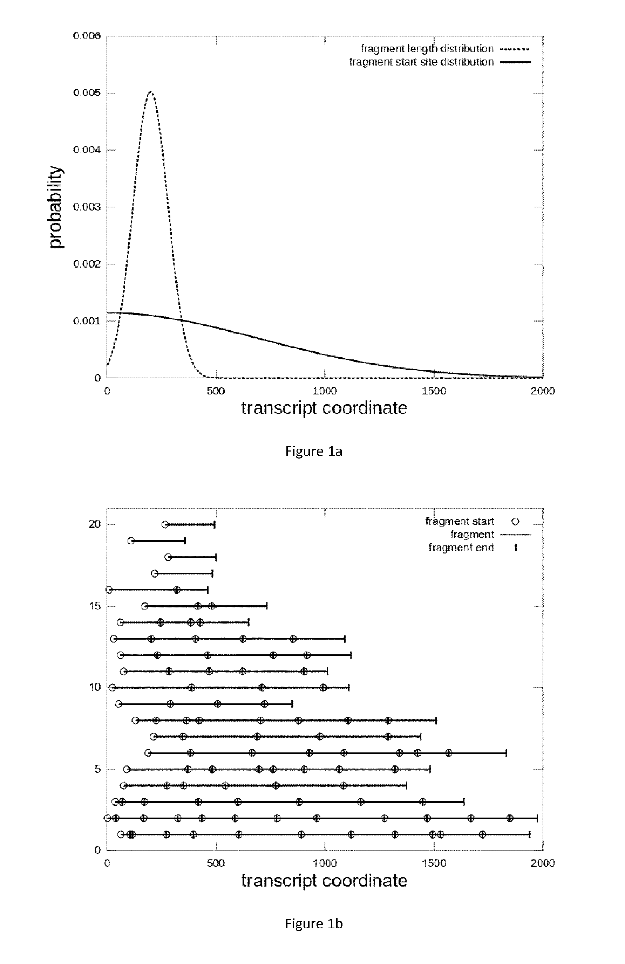

Figure 1: Fragment start site distribution with a strong 5' bias.

(a) Fragment start site distribution $p(s)$ and fragment length distribution $p(l|s = 1)$ for transcript $t_1$. The pdf $p(s)$ is a Gaussian with zero mean and standard deviation 700, whereas $p(l|s = 1)$ is a Gaussian with mean 200 and standard deviation 80.

Both $p(s)$ and $p(l|s = 1)$ are normalized such that $\sum_{s=1}^{2000} p(s) = 1$ and $\sum_{l=1}^{2000} p(l \mid s = 1) = 1$.

(b) Typical fragment sequences generated by fragmenting single copies of transcript $t_1$.

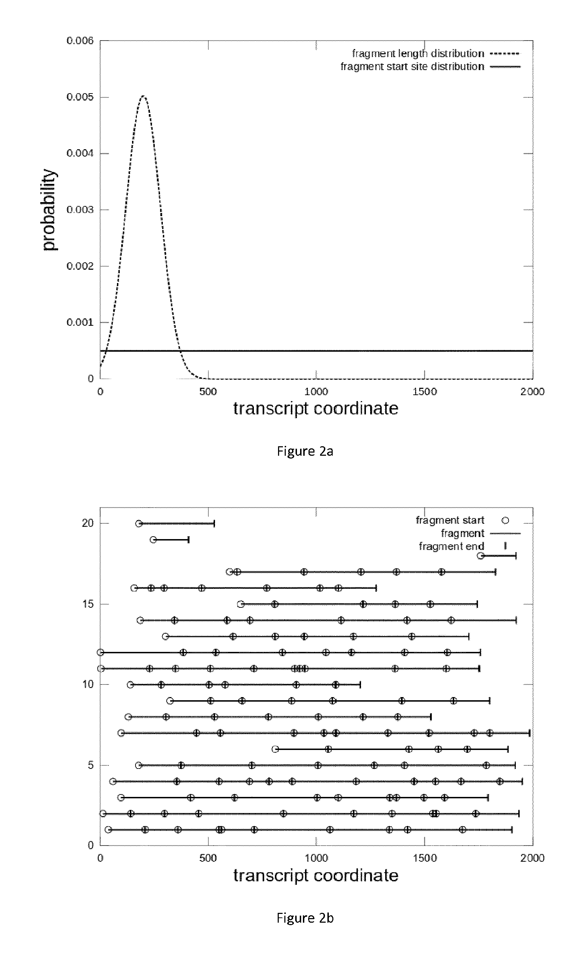

Figure 2: Uniform fragment start site distribution.

(a) Fragment start site distribution $p(s)$ and fragment length distribution $p(l|s=1)$ for transcript $t_2$. The pdf $p(s)$ is a uniform distribution whereas $p(l|s=1)$ is a Gaussian with mean 200 and standard deviation 80. Both $p(s)$ and $p(l|s=1)$ are normalized such that $\sum_{s=1}^{2000} p(s) = 1$ and $\sum_{l=1}^{2000} p(l \mid s = 1) = 1$.

(b) Typical fragment sequences generated by fragmenting single copies of transcript $t_2$.

Figure 3: Fragment sequence synthesis (FSS).

Figure 4: Different types of fragmentation.

Figure 5: Fragment sequence reconstruction (FSR).

Figure 6: Common features of the FSS and FSR.

Figure 7: 50 fragment assemblies with equidistant assembly id's from the total 217,697 fragment assemblies obtained for 1,000,000 fragments sampled from the fragment distribution $p(r|t=i)$ derived from the pdf's in Figure 1a.

Figure 8: Number of fragments for the 50 assemblies in Figure 7.

Figure 9: Histogram of the number of fragments in the complete set of 217,697 assemblies obtained for the 1,000,000 fragments sampled from the distribution $p(r|t=i)$ derived from the pdf's in Figure 1a.

Figure 10: Estimates of $p(f=m|t=i,c=1)$ for the fragment distribution $p(r|t=i)$ derived from the pdf's in Figure 1a and different sample sizes.

Figure 11: Estimates of $p(f=m|t=i,c=1)$ for the fragment distribution $p(r|t=i)$ derived from the pdf's in Figure 2a and different sample sizes.

Figure 12: 50 fragment assemblies with equidistant assembly id's from the total 209,126 fragment assemblies obtained for 1,000,000 fragments sampled from the fragment distribution $p(r|t=i)$ derived from the pdf's in Figure 1a with a window size of 15 base pairs. In comparison to Figure 7 the position after the fragment end does not necessarily coincide with the start of the subsequent fragment in the assembly.

Figure 13: Estimates of $p(f=m|t=i,c=1)$ for uniform $p(s|t=i)$ and 1,000,000 fragment samples with different windows around assembly start and end.

Figure 14: Estimates of $p(f=m|t=i,c=1)$ for uniform $p(s)$, a window size of 15 base pairs and different fragment sample sizes.

Figure 15: Estimates of $p(f=m|t=i,c=1)$ for non-uniform $p(s|t=i)$ and 1,000,000 fragment samples with different windows around assembly start and end.

Figure 16: Estimates of $p(f=m|t=i,c=1)$ for non-uniform $p(s)$, a window size of 15 base pairs and different fragment sample sizes.

Figure 17: Approximation of $p(c=n|t=i,f=m)$ with the central limit theorem for non-uniform $p(s|t=i)$ and $m=25$ fragments.

Figure 18: 90% confidence intervals for estimates of $p(c=n|t=i,f=m)$ for Gaussian and uniform $p(s|t=i)$.

Figure 19: Fragment length distribution in NGS experiment.

Figure 20: FPKM values for seven ERCC transcripts. The correlation between the number of molecules and the FPKM value is 0.8128. The linear regression model without intercept has slope 0.3483. The linear regression model with intercept has slope 0.6167 and intercept -2.0332.

Figure 21: Length adjusted FPKM values for seven ERCC transcripts. The correlation between the number of molecules and the length adjusted FPKM value is 0.7771. The linear regression model without intercept has slope 0.3610. The linear regression model with intercept has slope 0.5605 and intercept -1.5111.

Figure 22: ERCC00130 fragment start distribution in transcript coordinates.

Figure 23: ERCC00074 fragment start distribution in transcript coordinates.

Figure 24: ERCC00002 fragment start distribution in transcript coordinates.

Figure 25: 75 randomly drawn assemblies for ERCC00130. The complete set of assemblies was generated by the FSS from 100,000 fragments sampled from the length distribution in Figure 19 and the start site distribution 21 with a window size of 15 base pairs. This resulted in a total of 56,272 assemblies. The assemblies in this figure contain 125 fragments.

Figure 26: 75 randomly drawn assemblies for ERCC00074. The complete set of assemblies was generated by the FSS from 100,000 fragments sampled from the length distribution in Figure 19 and the start site distribution 22 with a window size of 15 base pairs. This resulted in a total of 57,769 assemblies. The assemblies in this figure contain 130 fragments.

Figure 27: 75 randomly drawn assemblies for ERCC00002. The complete set of assemblies was generated by the FSS from 100,000 fragments sampled from the length distribution in Figure 19 and the start site distribution 22 with a window size of 15 base pairs. This resulted in a total of 49,332 assemblies. The assemblies in this figure contain 178 fragments.

Figure 28: Distributions of number of fragments within assemblies for ERCC00002, ERCC00074 and ERCC00130.

Figure 29: Estimated number of molecules for seven ERCC transcripts. The correlation between the number of molecules and the estimated number of molecules is 0.8792. The linear regression model without intercept has slope 0.3620. The linear regression model with intercept has slope 0.9462 and intercept -4.4253.

**Examples:**

**Example 1.1: Calculating transcript concentration from transcript abundance by fragment sequence synthesis**

One of the main purposes of next generation sequencing (NGS) is to determine the concentration, i.e. the number of copies, of a preselected nucleic acid of interest, such as a transcript within an mRNA sample, which is used for representative purposes only in the examples. Of course any example can be performed with any nucleic acid of interest. Concentration determination is typically done by mapping the reads (which are essentially fragments defined by their nucleic acid sequence) from an NGS experiment onto a reference genome and normalizing the number of reads that mapped within the definition of the transcripts. The intention of the normalization is to obtain a value which is directly correlated to the concentration of the transcripts and which is identical for transcripts with equal concentration. One of the most common normalization methods results in the so-called "fragments per kilo base per million reads" (FPKM) measure. For a given transcript $t$, this measure divides the number of reads $r(t)$ that map within the definition of transcript $t$ by the total number of reads in the NGS experiment $|R|$ and multiplies by one million. This value, which is interpreted as the expected value of reads that map within the definition of the transcript for an NGS experiment with a total number of one million reads, is then divided by the length of the transcript $l(t)$ and multiplied by 1000. Hence the FPKM measure of transcript $t$ is given by

$$FPKM(t) = \frac{r(t)10^9}{|R|l(t)} \qquad (1)$$

Dividing by the length of the transcript $l(t)$ in (1) accounts for the fact that longer transcripts generate more reads and thus their read counts are higher than those of shorter transcripts with identical concentration. However, for two transcripts $t_1$ and $t_2$ of different length but same concentration their *FPKM* values are identical only if the following holds

$$\frac{l(t_2)}{l(t_1)}r(t_1) = r(t_2) \qquad (2)$$

This implies that in order for the *FPKM* measure to be a proper measure of transcript concentration the number of reads $r(t)$ has to be linearly correlated to the length of the transcript $l(t)$ and that for constant concentration the length $l(t)$ is the only factor that influences the number of reads $r(t)$. This is, however, an unrealistic assumption if one takes into consideration that each fragment originates from the fragmentation of a single copy of a transcript and thus from a sequence of fragments which obeys certain constraints. One such constraint is that the start of a fragment does not occur before the end of the previous fragment in the sequence. Another constraint is that the probability distribution of the fragments is given by $p(r|t=i)$. Consider, for instance, two transcripts $t_1$ and $t_2$ with the same length $l(t_1)=l(t_2)$ of 2000 base pairs, where the fragment start sites of transcript $t_1$ have a strong 5' bias, as shown by the solid line in Figure 1. In addition, the dashed line in Figure 1 shows the distribution of the fragment lengths for fragments starting at the first base pair. For fragments whose start position s is not at the first base pair the distribution of the fragment lengths $p(l|t=i,s)$ is derived from the dashed line in Figure 1 by restricting this distribution to all possible values $l=1,...,2000-s+1$ and renormalizing the sum to one. The fragment start site and length distributions in Figure 1 are renormalized Gaussians with means 0 and 200 and variances 700 and 80 whose sum over all the 2000 base pairs equals one. In comparison to transcript $t_1$, the fragment start positions for transcript $t_2$ are evenly distributed, as can be seen from the solid line in Figure 2. The distribution of the fragment lengths for $t_2$ for fragments starting at the first base pair is again a renormalized Gaussian with mean 200 and variance 80. Assume furthermore that fragmentation of a single copy of the transcripts generates fragment sequences such that the start of a fragment follows immediately after the end of the previous fragment in the sequence. Under this assumption typical fragment sequences generated for single copies of transcripts $t_1$ and $t_2$ are shown in Figures 1b and 2b. Each fragment in these figures is depicted by a horizontal line which is bordered by a circle and a short vertical bar. The center of the circle and the bar represent the start and end of the fragment, respectively. Thus the full horizontal lines in Figures 1b and 2b represent sequences of fragments. The fact that the start of a fragment follows immediately after the end of the previous fragment is indicated by the close proximity of circles and vertical bars in Figures 1b and 2b. The 5' bias of the fragment start site distribution of transcript $t_1$, on the other hand, is reflected by the fact that few fragment sequences for $t_1$ extend over the full length of $t_1$. Hence fragment sequences for transcript $t_1$ contain on average half as many fragments as the fragment sequences for transcript $t_2$. Thus if $t_1$ and $t_2$ have the same concentration, i.e. the same number of copies, transcript $t_1$ generates on average half as many fragments as transcript $t_2$. On the other hand, if $t_1$ and $t_2$ generate the same number of fragments the concentration of transcript $t_1$ is about twice as high as the concentration of transcript $t_2$. This contradicts the FPKM measure which is identical for $t_1$ and $t_2$ in this case and therefore suggests that the concentration of $t_1$ and $t_2$ is the same. This example shows that the length of a transcript is not the only factor that influences the number of fragments generated from a transcript but that the fragment distribution $p(r|t=i)$ and other properties of the fragment sequences generated from a single copy of the transcript, such as their connectedness, play an important role as well.

Commonly used concentration measures, such as the *FPKM* measure, do not have any intuitive meaning by themselves but are abstract values which are somehow correlated to the concentration, i.e. the number of copies, of a transcript. In contrast, the method developed in this section directly calculates the probability $p(c=n|t=i)$ that n copies of transcript $t=i$ are present in an mRNA sample. The probability $p(c=n|t=i)$ can be factorized as follows.

$$p(c=n \mid t=i) = \sum_f p(c=n \mid t=i, f=m) p(f=m \mid t=i) \qquad (3)$$

Here $p(f=m|t=i)$ is the probability of observing $m$ fragments given that the transcript is $t=i$ and $p(c=n|t=i,f=m)$ is the probability of observing $n$ copies of transcript t=i given that $m$ fragments were produced by transcript $t=i$. The probability $p(f=m|t=i)$ is the distribution over the abundances of transcript t=i, which is provided by abundance estimating methods such as Cufflinks. Some of these methods return a single maximum likelihood estimate $\alpha_i$ for the abundance of transcript t=i in which case $p(f=\alpha_i|t=i)=1$ and $p(f=m|t=i)=0$ for $m \neq \alpha_i$. Since $p(f=m|t=i)$ is provided by already existing tools the goal of the method described here is the calculation of $p(c=n|t=i,f=m)$. Applying Bayes formula yields

$$p(c=n \mid t=i, f=m) = \frac{p(f=m \mid t=i, c=n) p(c=n)}{\sum_{n'=1}^{N} p(f=m \mid t=i, c=n') p(c=n')} \qquad (4)$$

Here $N$ is the maximal possible number of copies of $t=i$ and $p(c=n)$ is the prior knowledge about the number of copies of transcript $t=i$. If no prior knowledge is available then (4) reduces to

$$p(c=n \mid t=i, f=m) = \frac{p(f=m \mid t=i, c=n)}{\sum_{n'=1}^{N} p(f=m \mid t=i, c=n')} \qquad (5)$$

Assuming that the fragmentation of n copies of transcript $t=i$ is the repetition of n independent identically distributed processes yields

$$p(m_1, \ldots, m_n \mid t=i, c=n) = \prod_{j=1}^{n} p(f=m_j \mid t=i, c=1) \qquad (6)$$

And the probability of observing $m$ fragments in total generated by n copies of transcript t=i is therefore given by

$$p(f=m \mid t=i, c=n) = \sum_{m=m_1+\ldots+m_n} \prod_{j=1}^{n} p(f=m_j \mid t=i, c=1) \qquad (7)$$

Thus the main problem in estimating $p(c=n|t=i,f=m)$ is the estimation of $p(f=m|t=i,c=1)$ and the efficient calculation of $p(f=m|t=i,c=n)$ from $p(f=m|t=i,c=1)$. In order to obtain a good estimate for $p(f=m|t=i,c=1)$ it is necessary to find fragment sequences which resemble those generated by fragmenting a single copy of transcript $t=i$. Such fragment sequences, which are illustrated in Figures 1b and 2b, will be called single copy fragment sequences (SCFS's) in the following. Once a sufficiently large number of SCFS's has been found $p(f=m|t=i,copies=1)$ can be derived by accumulating the statistics of the number of fragments in these typical fragment sequences.

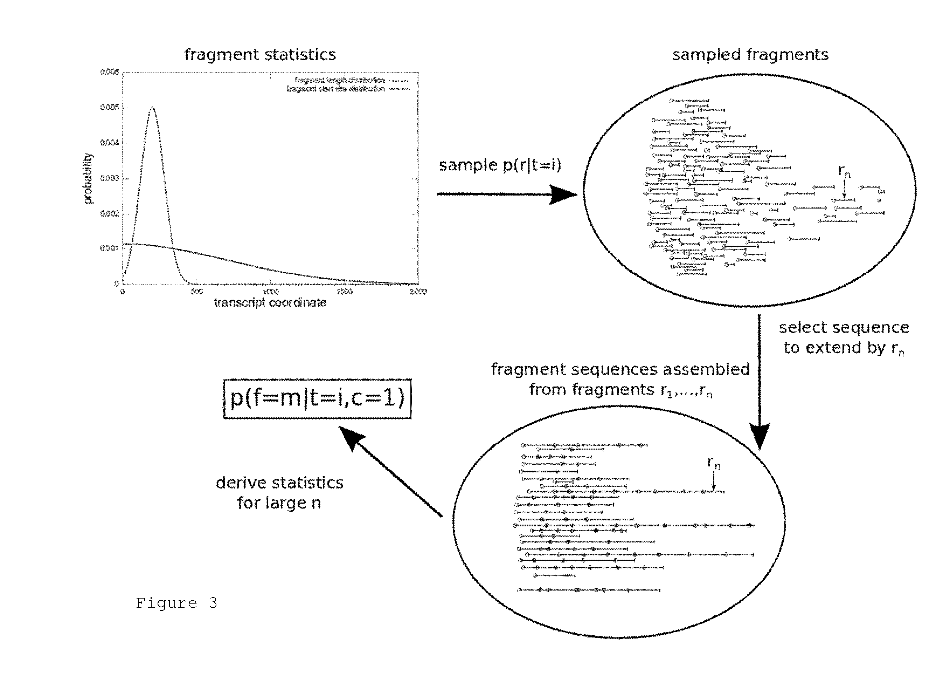

As mentioned in the discussion of Figures 1b and 2b SCFS's have to obey a number of constraints. One of them is that the fragments generated by fragmenting single copies of transcript $t=i$ are distributed according to $p(r|t=i)$. The distribution $p(r|t=i)$ can, for instance, be inferred from the data of an NGS experiment by the method described in European patent application EP 13175774.2. In addition SCFS's have to satisfy certain constraints regarding their topology. As mentioned before, the minimal topological constraint is that the start of a fragment does not occur before the end of the previous fragment in the sequence. However, some fragmentation might, as shown in Figures 1b and 2b, produce SCFS's which follow more stringent constraints. In contrast to the distribution of the fragments, $p(r|t=i)$, it is currently not possible to infer the topological constraints of the SCFS's and it is therefore necessary to take an educated guess. The description of SCFS's via their constraints suggests to assemble samples of $p(r|t=i)$ into sequences which satisfy the topological constraints of the SCFS's in order to obtain fragment sequences which model the statistics of the SCFS's. This procedure, which will be called fragment sequence synthesis (FSS), is visualized in Figure 3. Here fragments are sampled from the fragment distribution $p(r|t=i)$ in the top left corner of Figure 3. The first $n$ sampled fragments can be seen in the right upper oval of Figure 3 with the $n$-th fragment $r_n$ highlighted. For $r_n$ the FSS has to select one of the fragment sequences generated from the first $n$-1 fragments, which is extended by $r_n$. The extended fragment sequence has to fulfill the topological constraints of the SCFS's. If no such extension by $r_n$ exists then $r_n$ forms a new sequence consisting only of itself. If, on the other hand, more than one extension by $r_n$ is possible then the FSS has to decide which fragment sequence to extend. This includes the possibility of merging several fragment sequences if they can be linked by $r_n$. Thus in addition to the fragment distribution $p(r|t=i)$ and the topological constraints of the SCFS's the FSS requires a procedure for choosing between multiple extension possibilities. If no preference is given to the extension of any particular fragment sequence then each fragment sequence can be extended with equal probability. Hence the process of selecting the fragment sequence to extend is probabilistic. After a sufficient number of iterations of the FSS, e.g. when a certain number of fragment sequences have been generated, the FSS terminates and the distribution of the number of fragments in the SCFS's, $p(f=m|t=i,c=1)$, is estimated from the generated fragment sequences.

**Example 1.2 Optimal fragment sequence reconstruction**

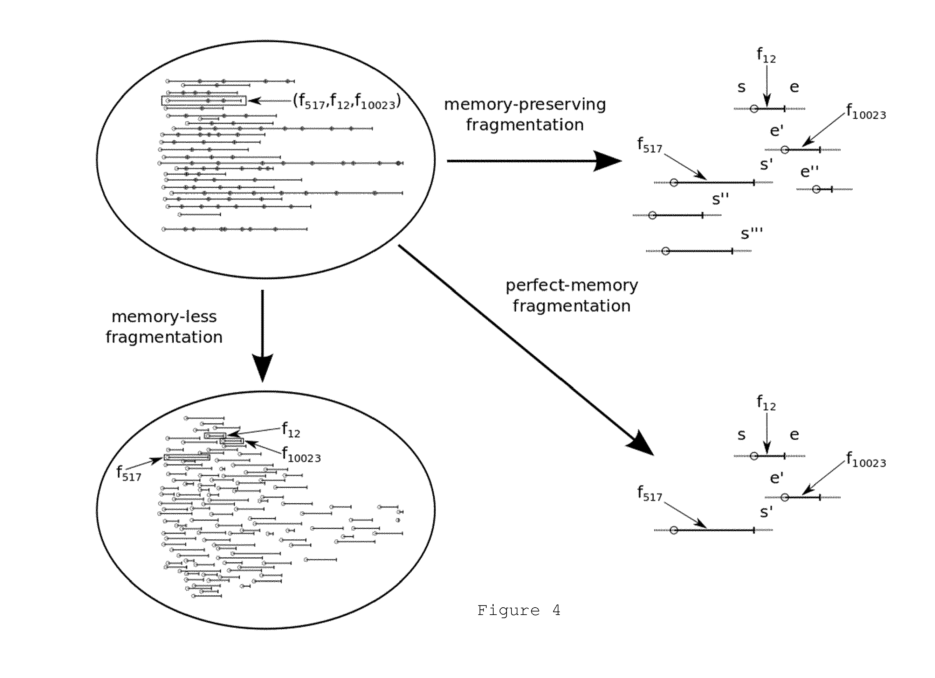

In current NGS experiments the single copy fragment sequences (SCFS's) of transcript $t=i$ are not recorded. Therefore, only the statistics $p(r|t=i)$ of the fragment locations irrespective of their SCFS's can be inferred from the data. Thus in current NGS experiments $p(r|t=i)$ forms the basis of the estimate of the abundance of transcript $t=i$. In the future, however, one can envisage that some information might be retained about the SCFS's of transcript $t=i$ and that it will become possible to reconstruct the SCFS's of transcript $t=i$. An important consequence of the ability to reconstruct the SCFS's of transcript $t=i$ is that it allows to count the copies of transcript $t=i$ and therefore provides an exact value of the concentration of transcript $t=i$. An SCFS might, for instance, be recorded if each fragment of an SCFS has connectors at its start and end, where the connector at the end of one fragment fits to the connector at the start of the successive fragment in the SCFS. Here the connectors can be part of the sequence of transcript $t=i$ or sequences artificially inserted at the start and end of a fragment. This situation is depicted on the right side of Figure 4, the left upper oval of which shows the SCFS's of transcript $t=i$. The highlighted SCFS consists of fragments $f_{517}, f_{12}$ and $f_{10023}$, where the subscript numbers are given to the fragments by the NGS sequencer and are therefore not correlated to the SCFS. The fragmentation in the left lower oval is memory-less. No information about the SCFS's is present in the set of fragments, which therefore corresponds to the fragmentation in current NGS experiments. The fragmentation in the right upper corner of Figure 4, on the other hand, shows a memory-preserving fragmentation. Here the fragments have connectors preceding their start and following their end. Fragment $f_{12}$ has connectors $s$ and $e$ before its start and end, respectively. Connector $s$ fits to the connectors $s'$, $s''$ and $s'''$ and therefore fragment $f_{12}$ can be preceded by the fragments which are followed by these connectors. There are three fragments in Figure 4 which are potential candidates among them the correct fragment $f_{517}$. Likewise, the connector e after the end of fragment $f_{12}$ fits to connectors $e'$ and $e''$ and the fragments which are preceded by these connectors are therefore potential successors to fragment $f_{12}$, among them the correct fragment $f_{10023}$. In the memory-preserving fragmentation in the right upper corner of Figure 4 the sequence of fragments $f_{517}, f_{12}, f_{10023}$ cannot be uniquely determined from the connectors alone and thus additional information about the fragments, such as their location on a reference genome, is needed to resolve ambiguities. In contrast, the fragmentation in the right lower corner of Figure 4 contains all the information needed to reassemble the correct SCFS's and can therefore be considered to be a perfect-memory fragmentation.

A simplified version of a memory-preserving fragmentation, which partially implements the concept of start and end connectors, can be realized by isolating individual copies of transcript $t=i$, for instance by applying a limiting dilution. After isolation a single barcode is ligated to the start of each fragment, which uniquely identifies the copy of transcript

$t=i$. Then the sequence of fragments generated by fragmenting a single copy of transcript $t=i$ can be reconstructed by aligning fragments with the same barcode to a reference genome, which resolves the order of the fragments within the sequence.

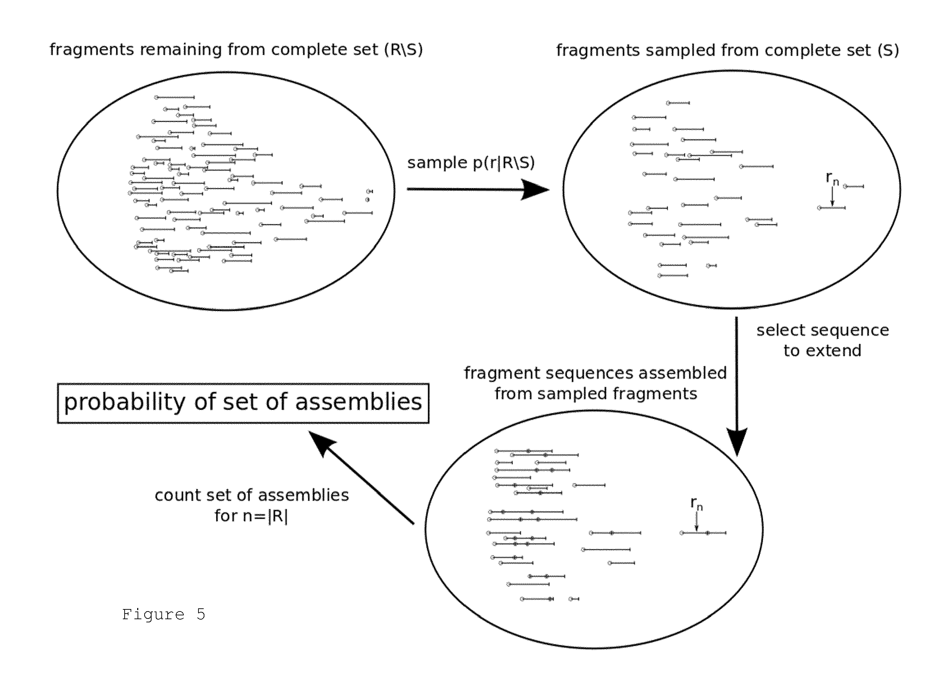

In general, due to inaccuracies in the data multiple reconstructions of the SCFS's from the fragments generated by a memory-preserving or even perfect-memory fragmentation will be possible and the reconstruction with the highest quality has to be selected. Figure 5 shows a method which estimates the probability of each SCFS reconstruction under a certain stochastic model and can therefore be used to evaluate the quality of SCFS reconstructions according to a maximum likelihood principle. This method will be called the fragment sequence reconstruction (FSR). Figure 5 shows the state of the FSR after the $n$-th iteration. From the original set of fragments which is the same as the one in the lower left oval in Figure 4, and which is denoted by $R$, $n$ fragments have been drawn randomly. The set of remaining fragments which is denoted by $R\backslash S$ is shown in the left upper oval in Figure 5. The set of the $n$ fragments already drawn from $R$ is contained in the right upper oval and is denoted by $S$ while the $n$-th fragment drawn from $R$ is denoted by $r_n$. The right lower oval in Figure 5 shows the fragment sequences generated from the fragments $r_1,...,r_{n-1}$ and the sequence which is extended by $r_n$. As previously, only sequences can be extended by $r_n$ such that the resulting sequence satisfies the topological constraints of the SCFS's and furthermore the FSR has to have a method to choose between different extension possibilities. Figure 5 shows that fragment sampling and sequence extension are performed iteratively until the last fragment in $R$ has been drawn, which corresponds to $n=|R|$. The count of the generated set of sequences is then increased by one and the whole procedure starts again from the full set of fragments $R$. After a sufficiently large number of repetitions the counts of the sets of fragment sequences are normalized by the total number of repetitions which gives an estimate of the probability of the sets of fragment sequences which can be generated from the complete set of fragments $R$. Subsequently, the set of fragment sequences with the highest probability can be chosen as the best reconstruction of the set of fragments $R$. Comparing Figures 3 and 5 shows that the fragment sequence synthesizer (FSS) and the fragment sequence reconstruction (FSR) are essentially the same algorithm and differ only in their input and termination criteria. This suggests that both the FSS and the FSR should be formulated as special cases of a more general algorithm. This algorithm will be called the fragment assembly algorithm (FAA) and will be developed in section 2.

### *Example 2: Fragment assembly algorithm*

### **Example 2.1 Common features of the fragment sequence synthesis and reconstruction**

It has already been noted that the fragment sequence synthesis (FSS) introduced in section 1.1 and the fragment sequence reconstruction (FSR) introduced in section 1.2 share many common features. It is the purpose of this section to describe those features and to develop a general framework which contains the FSS and FSR as special cases.

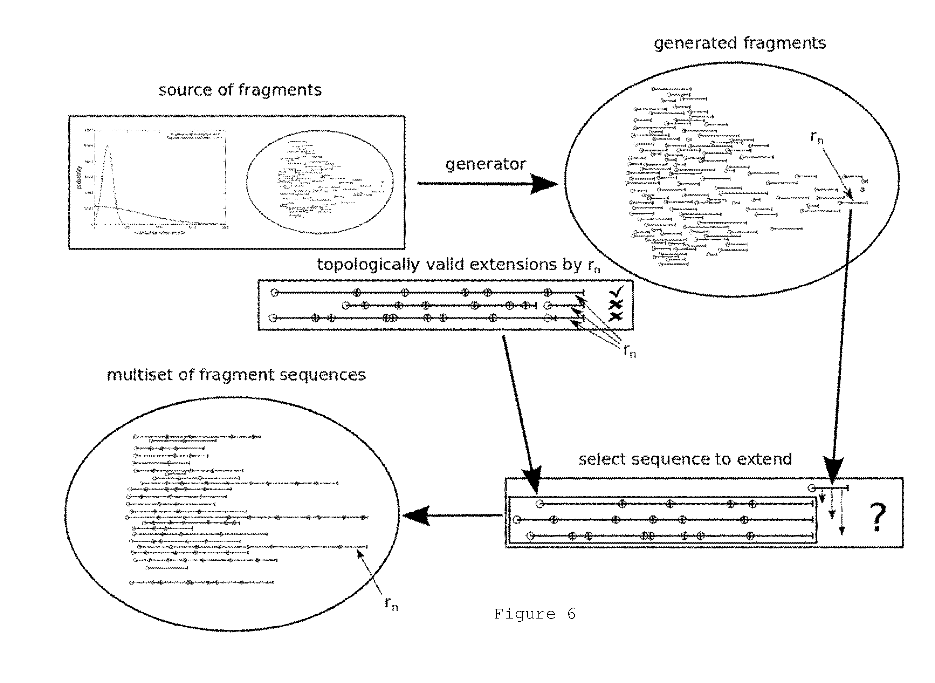

As indicated by Figure 6 both the FSS and FSR are iterative procedures that generate a set or, more precisely, a multiset of fragment sequences at each iteration. In comparison to a set, a multiset can contain an element several times, which can be the case for the output of the FSS and FSR since the same fragment sequence can be generated during multiple iterations. Another similarity between the FSS and FSR is that both have a random source of fragments as visualized in the left upper corner of Figure 6. For the FSS these are the samples of the probability distribution $p(r|t=i)$, for the FSR in the $n$-th iteration these are samples from the multiset of fragments $R$ which have been generated by fragmentation of transcript $t=i$ and which do not belong to the set $S=\{r_1...,r_{n-1}\}$ of fragments which have been used by the FSR in the previous $n$-1 iterations. Hence at the $n$-th iteration the random source of the FSR is a probability distribution $p(r|t=i,r_1,...,r_{n-1})$. From the randomly drawn fragments both FSS and FSR generate fragment sequences. This is done by combining the fragment drawn in the $n$-th iteration $r_n$ with the fragment sequences generated from the fragments $r_1,...,r_{n-1}$ of earlier iterations. The selection of the fragment sequence to be extended by $r_n$ is itself a random process. The process of selecting the sequence to extend is visualized in the lower right box of Figure 6. The newly generated multiset of fragment sequences, on the other hand, is depicted in the lower left oval shape of this figure, which shows that fragment $r_n$ has been added to one of the previously generated fragment sequences. Finally, both the iterations of the FSS and FSR are performed until their termination criteria are fulfilled. For the FSS this criterion is that the probability distribution of the fragment sequence lengths $p(l|t=i)$ has converged, for the FSR this is the requirement that the probabilities of the multisets of the fragment sequences have converged. In summary, the FSS and FSR have the following list of features in common:

- FSS and FSR are iterative procedures with termination criteria;
- At each iteration FSS and FSR draw a random fragment $r_n$ from a distribution of fragments;
- At each iteration the FSS and FSR randomly select one or several of the already generated sequences to be extended by $r_n$.

As mentioned previously, the selection of the fragment sequence to be extended by $r_n$ has to generate a fragment sequence which obeys the topological constraints of the SCFS's. Hence the probability of selecting a fragment sequence such that extension by $r_n$ yields a fragment sequence which violates the topological constraints is zero. It is possible to arrive at such a selection criterion by defining the topological constraints and giving the same probability of selection to all fragment sequences whose extension by $r_n$ yields a sequence which obeys the topological constraints. Thus, as indicated in the central box in Figure 6, the third point in the list above can be replaced by the following:

- Definition of topological constraints;
- At each iteration randomly choose one of the topologically valid extensions by $r_n$.

**Example 2.2 Formal definition of the fragment assembly algorithm**

The previous section discussed the common features of the fragment sequence synthesis and the fragment sequence reconstruction. It is the purpose of this section to formalize these features in a mathematical framework which contains the FSS and FSR as special cases.

Fragments are elements of a set $F$ and are assembled by the fragment assembly algorithm (FAA) into sequences of fragments $a=(r_1,...,r_n)$, where $n{\geq}1$, $r_i \in F$ and the length of $a$ will be denoted by $l(a)$. The set of sequences that are generated by the FAA is denoted by $A(F)$ and its elements $a{\in}A(F)$ are called assemblies. For a fragment $r{\in}F$ and a sequence $a{\in}A(F)$ there might exist an extension of $a$ by $r$ into another sequence $b{\in}A(F)$. This defines a relation on $A(F)$ which will be denoted by $a \xrightarrow{r} b$. It is also possible that more than one sequence can be simultaneously extended by a fragment $r{\in}F$ into another sequence. This can, for instance, be the case if $a_1=(r_1,...,r_n)$ and $a_2 = (r'_1,...,r'_m)$ and r connects $a_1$ and $a_2$ as follows $(r_1,...,r_n,r,r'_1,...,r'_m) \in A(F)$. In general therefore, the relation $a \xrightarrow{r} b$ is defined between finite subsets $a{\subseteq}A(F)$ and $b{\in}A(F)$. There is a special sequence which is the empty sequence $a_\emptyset=()$. The empty r sequence is extendable by any fragment $r{\in}F$ and fulfills $a_\emptyset{\rightarrow}(r)$. Thus $(r)$ is the extension of the empty sequence $a_\emptyset$ by $r$. Since $l(a)>0$ for $a{\in}A(F)$, $a_\emptyset{\notin}A(F)$, which can also be stated by saying that the FAA does not produce empty sequences. If the FAA is supposed to discard certain fragments this can be achieved by keeping the state of the FAA fixed between successive iterations. Each iteration of the FAA generates a multiset of fragment sequences. In comparison to a set a multiset can contain the same element multiple times and is therefore specified by its elements and their multiplicity. A multiset of assemblies can therefore be written as follows

$$A = \{(a, m_A(a)) : a \in A(F), m_A(a) \in \mathbb{Z}_0\} \qquad (8)$$

where $m_A(a)$ is the multiplicity of $a$. The set of all multisets of assemblies will be denoted by $AS(F)$. Each multiset of assemblies has an associated multiset of fragments which will be denoted by $F(A)$ and is given by

$$F(A) = \left\{ \left( r, \sum_{a \in A : r \in a} m_A(a) \right) : r \in F \right\} \qquad (9)$$

Thus the multiplicity of $r \in F$ in $F(A)$ is the number of times the r fragment occurs in assemblies $a \in A$. For $a \xrightarrow{r} b$ it is assumed that

$$F(b) = F(a) \cup r \qquad (10)$$

r Hence given $a,b \in A(F)$ such that $a \xrightarrow{r} b$ then fragment $r \in F$ is uniquely defined. The underlying set of a multiset $A$ will be denoted by $U(A)$ and is given by

$$U(A) = \{a \in A(F) : m_A(a) > 0\} \qquad (11)$$

If assemblies are drawn randomly with uniform distribution from a multiset of assemblies *A* then the probability of observing assembly $a \in U(A(F))$ is given by

$$p(a \mid A) = \frac{m_A(a)}{|A|} \qquad (12)$$

where $|A| = \sum_{a \in U(A)} m_A(a)$ is the number of elements in *A*. Similarly, the probability of observing an assembly of length *L* is given by

$$p(L \mid A) = \frac{\sum_{a \in U(A), l(a)=L} m_A(a)}{|A|} \qquad (13)$$

For $A \in AS(F)$ and $r \in F$ let $X(A,r)$ denote the multiset of extensions $a \xrightarrow{r} b$ where $a \in A$ unified with the empty assembly $a_\varnothing$.

$$X(A,r) = \left\{ (a \xrightarrow{r} b, m_{X(A,r)}(a \xrightarrow{r} b)) : a \subseteq U(A) \cup a_\varnothing, b \in A(F) \right\} \qquad (14)$$

The multiplicity $m_{X(A,r)}(a \xrightarrow{r} b)$ is the number of possibilities, including the multiplicity of *a*, $m_A(a)$, how *a* can be extended by *r* to yield *b*. Given *a* and *r* there might be more than one way to construct *b*. Consider, for instance, the sequences $a=(...,r_i,r,r_{i+1},...)$ and $b=(...,r_i,r,r,r_{i+1},...)$ then *b* can be derived by extension from *a* by inserting *r* immediately after $r_i$ or immediately before $r_{i+1}$. In the FAA's studied here, however, there is exactly one way how *a* can be extended by *r* to yield *b* and therefore

$$X(A,r) = \left\{ \left( a \xrightarrow{r} b, m_A(a) \right) : a \subseteq U(A), b \in A(F) \right\} \cup \left\{ \left( a_\varnothing \xrightarrow{r} (r), m_{X(A,r)}\left( a_\varnothing \xrightarrow{r} (r) \right) \right) \right\} \quad (15)$$

According to (12), the probability of a uniformly random extension $a \xrightarrow{r} b$ in $X(A,r)$ is given by

$$p\left( a \xrightarrow{r} b \mid X(A,r) \right) = \frac{m_{X(A,r)}\left( a \xrightarrow{r} b \right)}{|X(A,r)|} \qquad (16)$$

This probability will be called the canonical extension probability. Hence, if the extension probability is the canonical then

$$p\left( a \xrightarrow{r} b \mid A,r \right) = p\left( a \xrightarrow{r} b \mid X(A,r) \right) \qquad (17)$$

The probability in (16) is the extension probability of the FAA as defined in the previous section. The extension of the multiset of *assemblies A* via $a \xrightarrow{r} b$ will be denoted by $EXT(A, a \xrightarrow{r} b)$ and is given by the following multiset of assemblies

$$EXT\left(A, a \xrightarrow{r} b\right) = A \setminus a \cup b \qquad (18)$$

Thus the subset $a \subseteq A \cup a_\emptyset$ of the assemblies which are extended is removed from the multiset $A$ while the assembly $b$ that results from extension is added to the multiset. This implies

$$\left|EXT\left(A, a \xrightarrow{r} b\right)\right| = |A \setminus a| + 1 \qquad (19)$$

Since $a_\emptyset \notin A$ equation (18) implies

$$EXT\left(A, a_\emptyset \xrightarrow{r} (r)\right) = A \cup r \qquad (20)$$

and thus

$$\left|EXT\left(A, a_\emptyset \xrightarrow{r} (r)\right)\right| = |A| + 1 \qquad (21)$$

If $B$ can be derived from $A$ by extension then the following must hold

$$a = A \setminus B \subseteq A \cup a_\emptyset$$
$$b = B \setminus A \in A(F) \qquad (22)$$
$$r = F(B) \setminus F(A) \in F$$
$$B = EXT(A, a \xrightarrow{r} b)$$

Thus the extension $a \xrightarrow{r} b$ by which $B$ is derived from $A$ is unique and the extension probability can be uniquely defined on sets $A, B \in AS(F)$ as follows.

$$p\left(A \xrightarrow{r} B\right) = \begin{cases} p\left(a \xrightarrow{r} b\right) & A, B, r \ satisfy \ (22) \\ 0 & A, B, r \ do \ not \ satisfy \ (2222) \end{cases} \qquad (23)$$

If $A, B \in AS(F)$ satisfy (22) then by (12) and (19) the probability of observing assembly $x \in B$, $p(x \mid B)$, can be derived from the probability $p(x \mid A)$ as follows

$$p(x \mid B) = \begin{cases} \dfrac{p(x \mid A) \mid A \mid + 1}{\mid A \setminus a \mid + 1} & x = b \\[2mm] \dfrac{p(x \mid A) \mid A \mid - 1}{\mid A \setminus a \mid + 1} & x \in a \\[2mm] \dfrac{p(x \mid A) \mid A \mid}{\mid A \setminus a \mid + 1} & x \notin a \cup b \end{cases} \qquad (24)$$

Since $p\left( A \xrightarrow{r} B \right) = 0$ for fragments $r \in F$ which do not satisfy (22) the following holds

$$p(B \mid A) = \sum_{r \in F} p(r \mid A) \, p(A \xrightarrow{r} B) \qquad (25)$$

Since each $r \in F$ is extendable due to the presence of $a_\varnothing$ in $X(A,r)$ the following holds

$$\sum_{B \in AS(F)} p(B \mid A) = 1 \qquad (26)$$

and thus an FAA is a Markov chain on the countably infinite state space *AS(F)*. Since according to (12) and (13) every $A \in AS(F)$ has an associated probability distribution over the assemblies $a \in A$ and assembly lengths *L*, an FAA has associated hidden Markov models, where the observables are the assemblies $a \in A(F)$ and assembly lengths *l(a)*, respectively, and the hidden variables are the multisets of assemblies $A \in AS(F)$. Summing up the discussion so far, the fragment assembly algorithm (FAA) can be formally defined as follows.

Fragment assembly algorithm: A fragment assembly algorithm is a Markov chain which is defined by the following tuple

$$\left( F, p(r \mid r_1, \ldots, r_{n-1}), a \xrightarrow{r} b, p\left( a \xrightarrow{r} b \mid A, r \right) \right) \qquad (27)$$

where

- *F* is a set whose elements are called fragments
- $p(r \mid r_1, \ldots, r_{n-1})$ is a probability distribution on *F*, after observation of *n*-1 fragments, which is called the fragment probability. If the order of the sequence is irrelevant then $p(r_n \mid r_1, \ldots, r_{n-1}) = p(r \mid F(A))$. If the set of fragments previously observed is irrelevant then $p(r \mid A) = p(r)$.
- $a \xrightarrow{r} b$ is a relation defined between the set of finite subsets of *A(F)*, $2^{A(F)}$, and *A(F)* which indicates that $b \in A(F)$ is an extension of $a \subseteq A(F)$ by $r \in F$.
- $p(a \xrightarrow{r} b \mid A, r)$ is a probability distribution which is called the extension probability. $p(a \xrightarrow{r} b \mid A, r)$ is defined for each $A \in AS(F)$ and $r \in F$ on $a \subseteq A$, $b \in A$ and $r \in F$ and satisfies $p(a \xrightarrow{r} b \mid A, r) = 0$ for

$$a \xrightarrow{r} b \notin U(X(A,r)).$$

A special case of an FAA can be defined by the following tuple

$$\left( F, p\left(r \mid r_1,\ldots,r_{n-1}\right), a \overset{r}{\to} b, A(F) \right) \tag{28}$$

where

- $A(F)$ is a set of fragment sequences which are called assemblies

- The canonical extension probability in this case is given by

$$p\left( a \overset{r}{\to} b \mid A,r \right) = \frac{m_{X(A,r)}\left( a \overset{r}{\to} b \right)}{\mid X(A,r) \mid} \tag{29}$$

The state space of the fragment assembly algorithm is the countably infinite set $AS(F)$ and the probability of a transition from state $A \in AS(F)$ to state $B \in AS(F)$ is given by

$$p(B \mid A) = \sum_{r \in F} p(r \mid A)\, p(A \overset{r}{\to} B) \tag{30}$$

where $p\left( A \overset{r}{\to} B \right)$ is defined in (23). The initial state of the fragment assembly algorithm is given by

$$p(A) = \begin{cases} p(r) & A = \{(r)\} \\ 0 & \mid F(A) \mid > 1 \end{cases} \tag{31}$$

Thus for the initial state the probability of a multiset of assemblies $p(A)$ with a single underlying fragment $r$ equals the probability of the fragment $p(r)$ while $p(A)=0$ for sets of assemblies with more than one underlying fragment. An FAA gives rise to hidden Markov models where the observables are assemblies $a \in A(F)$ and assembly lengths $L=1,\ldots,N$ and the hidden variable is the multiset of assemblies $A \in AS(F)$.

The following examples show different sets of assemblies $AS(F)$, where $F$ is the set of intervals on a finite set of numbers.

**Example 2.3: Assemblies of non-overlapping fragments**

*Here a sequence of fragments a = $(r_1,\ldots,r_n)$ is an assembly a $\in A(F)$ if*

$$end(r_i) < start(r_{i+1}) - S \tag{32}$$

for $i=1,\ldots,n$-1 and some $S \geq 0$. Hence $a \overset{r}{\to} b \in X(A,r)$ means that $r$ can be inserted between successive elements of $a$ or alternatively before $r_1$ or after $r_n$ such that equation (32) is fulfilled for the resulting sequence $b$. A fragment $r$ connects two sequences $a_1 = (r_1\ldots,r_n)$ and $a_2 = (r_1',\ldots,r_m')$ and therefore $a_1, a_2 \overset{r}{\to} b$ if $end(r_n) < start(r)$-S and

$end(r) < start(r_1') - S$ . If $S$=0 then the assemblies are contiguous, since there are no gaps between successive fragments. The case $S=0$ will therefore be referred to as assemblies of contiguous fragments.

It is furthermore possible to express a preference for certain extensions $a \xrightarrow{r} b \in X(A,r)$ by modifying the canonical extension probability. This can, for instance, be done by weighting the canonical extension probability and subsequent renormalization. Such a weight might for instance indicate a preference for sequences where the fragment $r$ is far away from its neighbors in $b$, e.g. by weighting the canonical extension probability as follows

$$p(a \xrightarrow{r} b \mid X(A,r)) \frac{start(r) - end(r_i) + start(r_{i+1}) - end(r)}{2} \qquad (33)$$

with the necessary modifications for $r$ inserted before the start or after the end of $a$ and $a = (a_1, a_2)$.

**Example 2.4 Assemblies of potentially overlapping fragments**

*Here a sequence of fragments $a = (r_1,...,r_n)$ is an assembly $a \in A(F)$ if*

$$end(r_i) < start(r_{i+1}) + S \qquad (34)$$

for $i=1,...,n$-1 and some $S > 0$. The same observations, with the necessary modifications, as in the case of assemblies of non-overlapping fragments also apply here. In comparison, however, sequences of potentially overlapping fragments can contain the same fragment multiple times. The concept of assemblies of potentially overlapping fragments contradicts the minimal topological constraint mentioned before, which requires that the start of a fragment does not occur before the end of the previous fragment. It will, however, be seen to be convenient for computational purposes.

**Example 2.5 Assemblies of fragments with bounded distance**

*In this case a sequence of fragments $a=(r_1,...,r_n)$ is an assembly $a \in A(F)$ if*

$$\mid end(r_i) - start(r_{i+1}) \mid < B \qquad (35)$$

for $i=1,...,n$-1 and some $B > 0$. An assembly can, as in the previous example, contain the same fragment multiple times.
The above examples show that there exist many variants of assembly sets $A(F)$ and extension probabilities $p(a \xrightarrow{r} b \mid A,r)$ and therefore many variants of the FAA. It is furthermore easy to generate new FAA's from already existing FAA's by modifying their assembly sets $A(F)$ or extension probabilities $p(a \xrightarrow{r} b \mid A,r)$. Section 2.3 will employ FAA's with assemblies of contiguous fragments and will show that FAA's with assemblies of fragments with bounded distance can be used to efficiently approximate their fragment sequence synthesis.
Let $A_n$ denote the set of multisets $A \in AS(F)$ which can be generated in the $n$-th iteration of an FAA. Due to equation (31) and the fact that exactly one fragment is added to a multiset $A \in AS(F)$ in each iteration the following holds.

$$A_n = \{A \in AS(F) : \mid F(A) \mid = n+1\} \qquad (36)$$

Therefore $A_n \cap A_{n+1} = \emptyset$ and hence an FAA can be interpreted as a branching process with no overlap between successive generations. Let $A_n \in A_n$ then the following holds

$$p^{(n+1)}(A_{n+1}) = \sum_{A_n \in A_n} p^{(n)}(A_n) p(A_{n+1} \mid A_n) \qquad (37)$$

where $p^{(n)}(A_n)$ is the probability of multiset $A_n \in A_n$ after the $n$-th iteration. Equation (37) holds since $p^{(n)}(A)=0$ for $A \notin An$. Let $b \in A(F)$ be an assembly then the probability of $b$ in the $n$+1-th iteration is given as follows

$$p^{(n+1)}(b) = \sum_{A_{n+1} \in \mathcal{A}_{n+1}} p^{(n+1)}(A_{n+1}) p(b \mid A_{n+1}) \tag{38}$$

where $p(b|A_{n+1})$ is given by (12). Substituting (37) into (38) yields the following equation.

$$p^{(n+1)}(b) = \sum_{A_{n+1} \in \mathcal{A}_{n+1}} \sum_{A_n \in \mathcal{A}_n} p^{(n)}(A_n) p(A_{n+1} \mid A_n) p(b \mid A_{n+1}) \tag{39}$$

Due to (18), (20) and (12) $p(x|B)$ can be derived from $p(x|A)$ as follows.

$$p(x \mid B) = \begin{cases} \dfrac{p(x \mid A) \mid A \mid + 1}{\mid A \setminus a \mid + 1} & x = b \\[2ex] \dfrac{p(x \mid A) \mid A \mid - 1}{\mid A \setminus a \mid + 1} & x \in a \\[2ex] \dfrac{p(x \mid A) \mid A \mid}{\mid A \setminus a \mid + 1} & x \notin a \cup b \end{cases} \tag{40}$$

If $F$ is the set of intervals of a finite set of numbers then $l(a)$ is bounded for all $a \in A(F)$ and $|A(F)|<\infty$, $|A_n|\to\infty$ for $A_n \in \mathcal{A}_n$ and $n\to\infty$ and therefore

$$p(x \mid A_{n+1}) \to p(x \mid A_n) \, n \to \infty \tag{41}$$

Hence for $n\to\infty$ the right hand side of equation (39) converges to

$$p^{(n+1)}(b) \to \sum_{A_n \in \mathcal{A}_n} p^{(n)}(A_n) p(b \mid A_n) = p^{(n)}(b) \, n \to \infty \tag{42}$$

Let $p^{(n)}(L)$ denote the probability of observing an assembly $a \in A(F)$ of length $l(a)=L$ in the n-th iteration, then the following can be derived from equation (42).

$$p^{(n+1)}(L) \to \sum_{A_n \in \mathcal{A}_n} p^{(n)}(A_n) p(L \mid A_n) = p^{(n)}(L) \, n \to \infty \tag{43}$$

Equations (42) and (43) show that the difference between probabilities of successive iterations of the fragment sequence synthesis converges to zero. Thus any FAA whose termination condition is based on the difference between successive probabilities will eventually terminate. However, equations (42) and (43) are only a necessary but not sufficient condition for the convergence of the probabilities. In principle it is possible that the difference between successive probabilities becomes arbitrarily small but that the probabilities do not converge. This, however, is only possible if the probabilities are not monotonic, which beyond a certain iteration has not been observed in the experiments described here. In the following therefore if $|p^{(n+1)}(L)-p^{(n)}(L)|<\varepsilon$ for all $L$ and a certain $\varepsilon > 0$ then $p^{(n)}(L)$ will be interpreted as the distribution $p(f = L|t = i,c = 1)$.

**Example 2.6 Fragment sequence synthesis and numerical estimation of the distribution $p(f = m \mid t = i,c = 1)$**

Whereas the previous section discussed the theoretical aspects of the fragment assembly algorithm this section focuses on some practical aspects of the fragment sequence synthesis (FSS). In particular this section deals with the question how many sampled fragments are necessary in order to obtain a good estimate of $p(f = m \mid t = i,c = 1)$ and how the speed of the convergence of the FSS can be increased.

EP 2 868 752 A1

**Example 2.6.1 Assemblies of contiguous fragments**

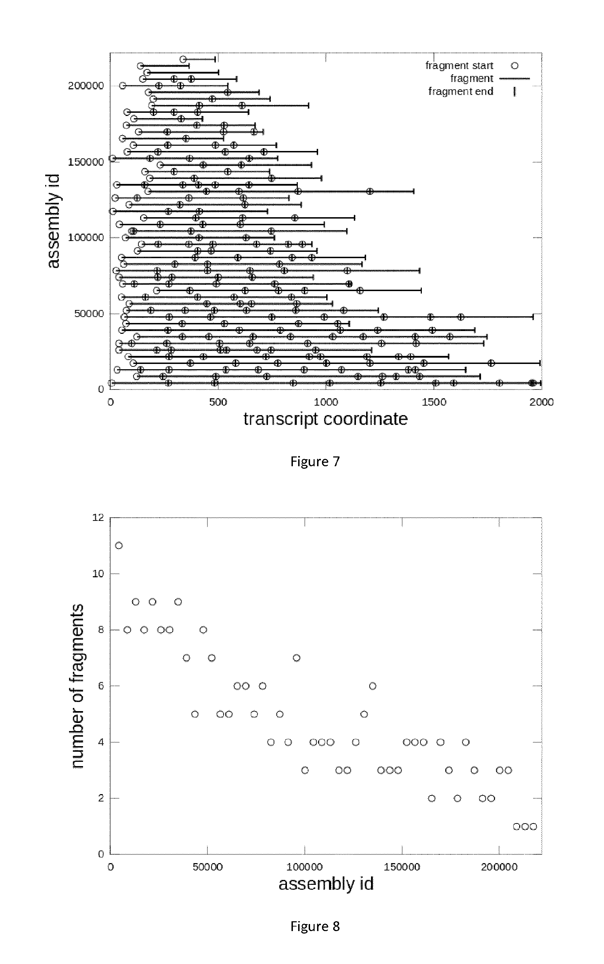

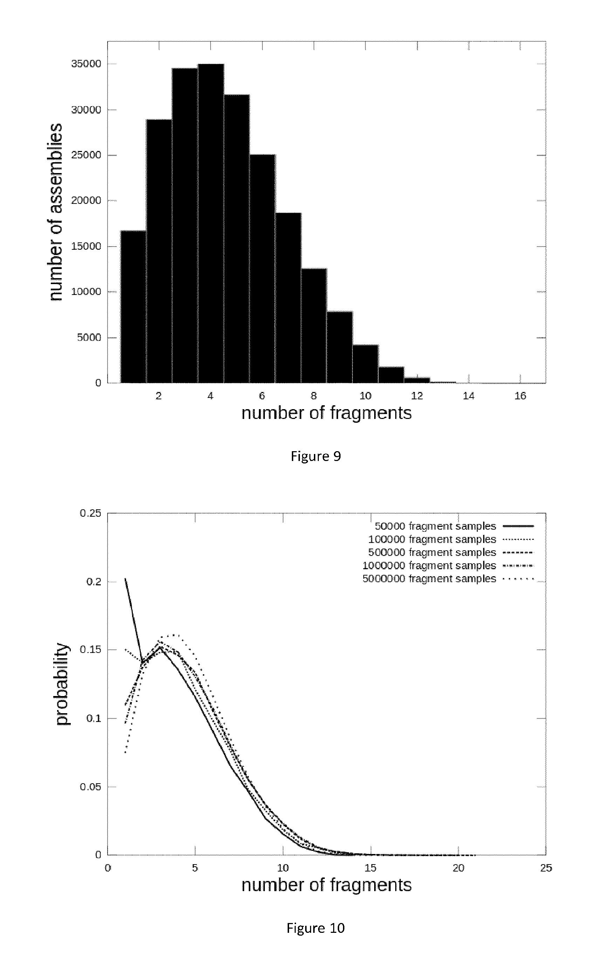

Consider the fragment distribution $p(r|t=i)$ whose fragment start site and length distributions are derived from the distributions in Figure 1a. The fragment start site distribution $p(s|t=i)$ is the Gaussian with mean 0 and standard deviation 700 in Figure 1a renormalized to the length of the transcript, which is 2000 base pairs long. The length distribution, on the other hand, of fragments starting at the first base pair is the renormalized Gaussian in Figure 1a with mean 200 and variance 80. Fragment sequences are considered to be assemblies if the start of a fragment follows immediately after the end of the previous fragment in the sequence, hence $end(r_i) + 1 = start(r_{i+1})$. The extension probability of the FSS synthesis is the canonical in this case. Figure 7 shows a selection of fragment assemblies that were generated by the FSS for a sample of 1,000,000 fragments which resulted in 217,697 assemblies in total. The x-axis in Figure 7 gives the transcript coordinate in base pairs, while the y-axis gives the assembly id which is the index of the assembly in the assembly list. Thus the assembly id equals 1 for the first assembly added to the assembly list in the FSS and equals the total number of assemblies for the last assembly added to the assembly list. For Figure 7, 50 assemblies were chosen with equidistant assembly id's starting at 4,354 and ending at 217,697. The circles in Figure 7 represent the start positions of the fragments, while the straight lines represent the length of the fragments starting at the start position to the left. Figure 7 shows that the distribution of the fragment start sites in the assemblies follows roughly the Gaussian with mean 0 and standard deviation 700 in Figure 1a, whereas the distribution of the fragment lengths follows roughly the Gaussian with mean 200 and standard deviation 80. Figure 7 shows also that the length of the fragment assemblies decreases with increasing assembly id, which is due to the fact that competition for extension increases between assemblies in later iterations of the FSS. This effect is also visualized in Figure 8 which shows the number of fragments in the assemblies depicted in Figure 7. Dividing the number of assemblies in the bins in Figure 9 by the total number of assemblies, i.e. 217,697, yields the estimate for $p(f=m|t=i,c=1)$ in Table 1.

Table 1: Estimate for $p(f=m|t=i,c=1)$ from a sample of 1,000,000 fragments yielding 217,697 fragment assemblies for the Gaussian $p(s|t=i)$ in Figure 1a.

| *m* | $p(f = m|t = i, c = 1)$ |
|---|---|
| 1 | 7.678562e-02 |
| 2 | 1.327304e-01 |
| 3 | 1.586241e-01 |
| 4 | 1.607372e-01 |
| 5 | 1.452110e-01 |
| 6 | 1.150958e-01 |
| 7 | 8.574303e-02 |
| 8 | 5.769028e-02 |
| 9 | 3.622007e-02 |
| 10 | 1.929287e-02 |
| 11 | 8.268373e-03 |
| 12 | 2.751531e-03 |
| 13 | 6.982182e-04 |
| 14 | 1.240256e-04 |
| 15 | 1.837416e-05 |
| 16 | 4.593541e-06 |
| 17 | 4.593541e-06 |

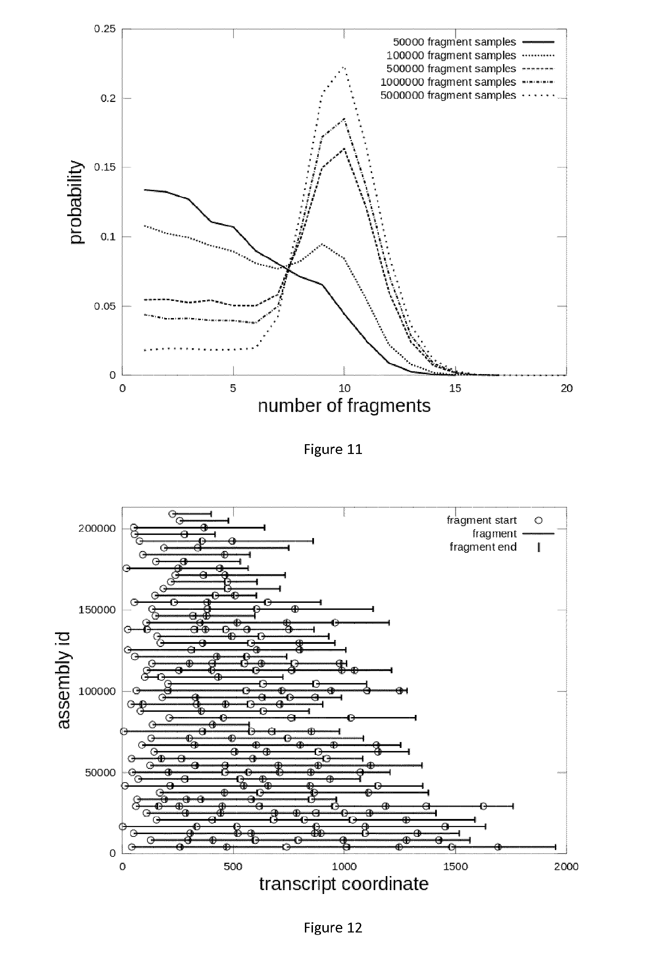

The estimation of $p(f=m|t=i,c=1)$ converges for the $p(r|t=i)$ derived from the distributions in Figure 1a with increasing sample size as can be seen from Figure 10. The size of the fragment sample necessary to obtain convergence depends on the nature of $p(r|t=i)$. Consider for instance the $p(r|t=i)$ which is derived from the distributions in Figure 2a where in comparison to Figure 1a the Gaussian for the fragment start site distribution has been replaced by a uniform distribution. The distribution $p(f=m|t=i,c=1)$ estimated for the $p(r|t=i)$ derived from Figure 2a for different sample sizes is visualized in Figure 11. For small sample sizes this figure shows the presence of a considerable number of short assemblies, which are between 1 and 6 fragments long. These assemblies are also present for larger sample sizes but are far less relative to the assemblies consisting of larger numbers of fragments. This suggests that the left tail of the estimate of $p(f=m|t=i,c=1)$ ultimately converges to zero for sample sizes beyond 5,000,000 fragments. Thus, in comparison to the $p(r|t=i)$ derived from the distributions in Figure 1a, a much higher number of fragment samples is necessary for the $p(r|t=i)$ derived from Figure 2a to obtain an estimate of $p(f=m|t=i,c=1)$ of comparable quality. This stems from the fact that in the FSS the assembly list is initially biased towards shorter assemblies which are therefore overestimated in early iterations of the algorithm. This negative effect on the convergence of the algorithm can be resolved by removing some of the short assemblies from the statistics. When removing assemblies from the assembly list it is important not to change the distribution of the fragment samples as this would result in an estimate of $p(f=m|t=i,c=1)$ for a fragment distribution different from $p(r|t=i)$. Removing short assemblies without removing any fragments from the fragment sample can be achieved by relaxing the conditions on what constitutes an assembly. This topic will be investigated in the following section.

**Example 2.6.2 Assemblies with bounded distance**

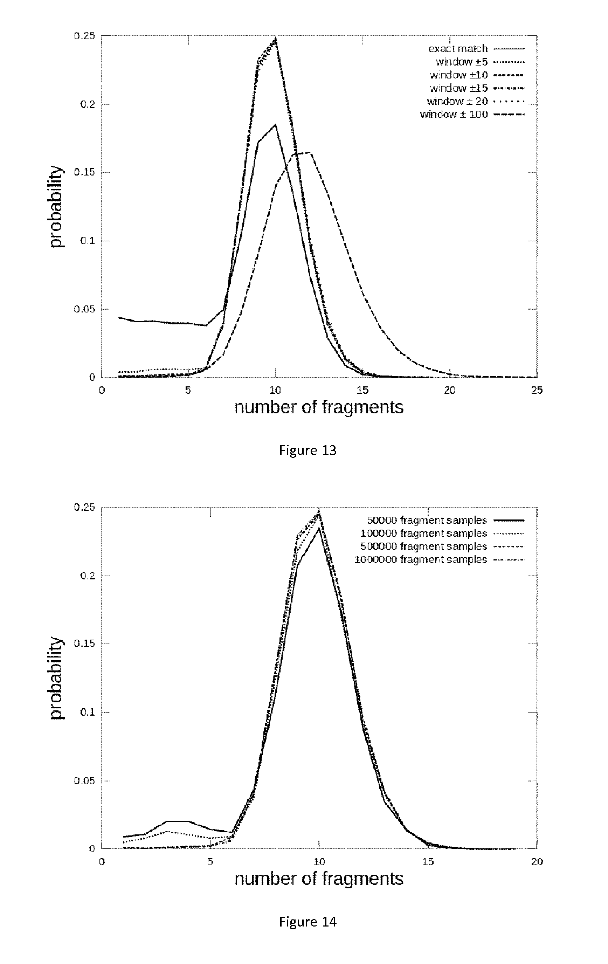

Instead of requiring an exact match at the start and end of fragments, i.e. $end(r_i)+1=start(r_{i+1})$, one can study assemblies of fragments with bounded distance, i.e. $|start(r_{i+1})-end(r_i)|\leq B$. This removes some of the short assemblies from the assembly list while retaining all the fragments in the fragment sample. Figure 12 shows 50 assemblies with equidistant assembly id's from the total 209,126 fragment assemblies obtained for 1,000,000 fragments sampled from the fragment distribution $p(r|t=i)$ derived from the pdf's in Figure 1a with a window size of 15 base pairs. In comparison to Figure 7 the position after the fragment end does not necessarily coincide with the start of the subsequent fragment in the assembly. Some adjacent fragments in Figure 12 are separated by gaps while other overlap, as can be seen by comparing the center of the circles with the location of the vertical bars. Figure 13 shows that the estimates for $p(f=m|t=i,c=1)$ based on 1,000,000 fragments sampled from the distribution derived from Figure 2a are not particularly sensitive to the value of $B$ as long as this value lies within a sensible range. On the other hand, convergence of the estimate is achieved for far fewer fragment samples than for assemblies of contiguous fragments as can be seen from Figure 14. In particular, the left tail of the estimated distribution is almost entirely removed for sample sizes above 500,000 fragments.

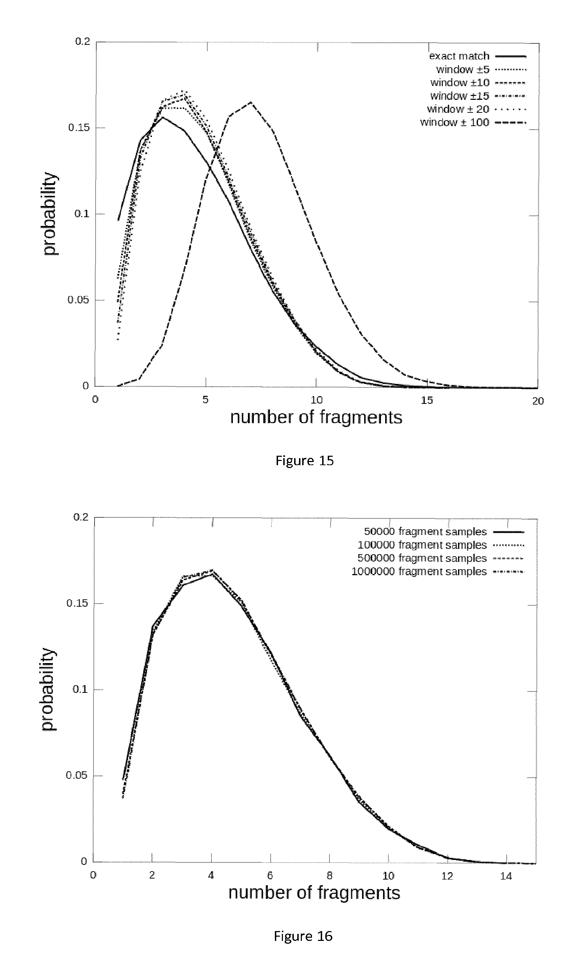

The same holds true for the Gaussian start site distribution. Figure 15 shows the estimates for $p(f=m|t=i,c=1)$ based on 1,000,000 fragment samples for window sizes ranging from 0 to 100, which indicates that the estimate of $p(f=m|t=i,c=1)$ is not very sensitive to the choice of the bound $B$. Figure 16, on the other hand, shows that for a window size of 15 the estimates for $p(f=m|t=i,c=1)$ converge for far fewer fragment samples than for assemblies of contiguous fragments. In particular, 100,000 fragment samples appear to be sufficient in order to achieve convergence for a window size of 15.

**Example 3: Estimating $p(c=n|t=i,f=m)$ from $p(f=m|t=i,c=1)$**

The estimation of $p(c=n|t=i,f=m)$ proceeds via the application of Bayes theorem in (5) and requires therefore the calculation of $p(f=m|t=i,c=n)$ in (7). While decomposing $m$ into all sums of $n$ summands might be possible for small $m$ it becomes increasingly impractical for larger $m$. In this case one can apply the central limit theorem which states that given the probability distribution $p(f=m|t=i,c=1)$, the distribution of the sum of $n$ copies $p(f=m|t=i,c=n)$ converges to a normal distribution for $n\rightarrow\infty$. The mean of this normal distribution is given by

$$\mu_n = n\mu_1 \qquad\qquad (44)$$

and the standard deviation is given by

$$\sigma_n = \sqrt{n}\sigma_1 \qquad\qquad (45)$$

where $\mu_1$ and $\sigma_1$ are the mean and standard deviation of $p(f=m|t=i,c=1)$. Thus the probability $p(f=m|t=i,c=n)$ converges as follows

$$p(f = m \mid t = i, c = n) \rightarrow \int_{m-\frac{1}{2}}^{m+\frac{1}{2}} \frac{1}{\sqrt{2\pi}\sigma_n} e^{-\frac{(x-\mu_n)^2}{2\sigma_n^2}} \, dx \; n \rightarrow \infty$$

$$(46)$$

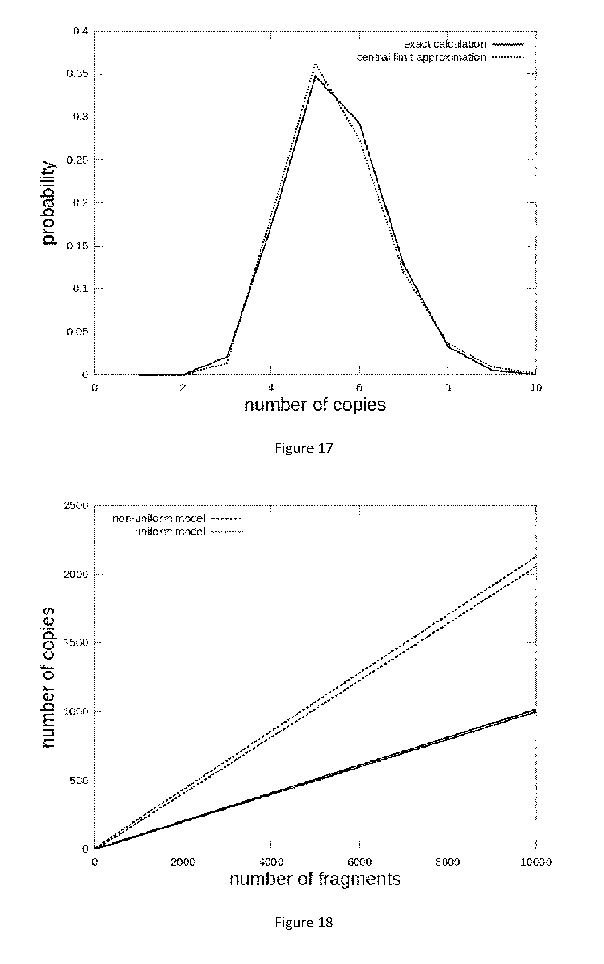

Figure 17 shows a comparison between the estimate of $p(c=n|t=i,f=m)$ based on the exact calculation of $p(f=m|t=i,c=n)$ and the approximation by the central limit theorem in 46 for the Gaussian $p(s|t=i)$ and $m$=25 fragments. This figure shows that both estimates of $p(c=n|t=i,f=m)$ are very similar and that the maximum likelihood estimate of the number of copies $n$ is the same in both cases. Table 2 gives the median and the 90% confidence interval for the distributions $p(c=n|t=i,c=m)$ estimated with the central limit theorem for Gaussian and uniform $p(s|t=i)$. The number of fragments $m$ ranges from 25 to 10000. The median in Table 2 is close to the number of fragments $m$ divided by the mean of the distribution $p(f=m|t=i,c=1)$. Hence dividing the number of fragments $m$ by the expected value of fragments for a single copy of a transcript can be used as a rule of thumb to estimate the number of copies of a transcript. Since the mean of the distribution $p(f=m|t=i,c=1)$ for the Gaussian $p(s|t=i)$ is close to 5 whereas it is close to 10 for the uniform $p(s|t=i)$ the expected value of copies for the same number of fragments is about twice as high for the Gaussian $p(s|t=i)$ than for the uniform $p(s|t=i)$.

Table 2: Medians and confidence intervals for the estimated $p(c=n|t=i,f=m)$ for varying $m$ and Gaussian and uniform $p(s|t=i)$.

| | Gaussian | | | uniform | | |
|---|---|---|---|---|---|---|
| m | % | median | 95% | 5% | median | 95% |
| 25 | 3 | 5 | 7 | 1 | 2 | 3 |
| 50 | 8 | 10 | 13 | 4 | 5 | 6 |
| 100 | 7 | 21 | 25 | 9 | 10 | 11 |
| 500 | 6 | 104 | 113 | 48 | 50 | 52 |
| 1000 | 98 | 209 | 221 | 98 | 100 | 103 |
| 5000 | 020 | 1045 | 1071 | 498 | 504 | 510 |
| 10000 | 2055 | 2091 | 2127 | 999 | 1008 | 1017 |

Figure 18 visualizes the 90% confidence intervals in Table 2 of the distributions $p(c=n|t=i,f=m)$ for the Gaussian and uniform $p(s|t=i)$ and different numbers of fragments $m$. This shows that the confidence intervals are very narrow and that the estimated number of copies $n$ is therefore reliable.

**Example 4: Experiments on NGS data**

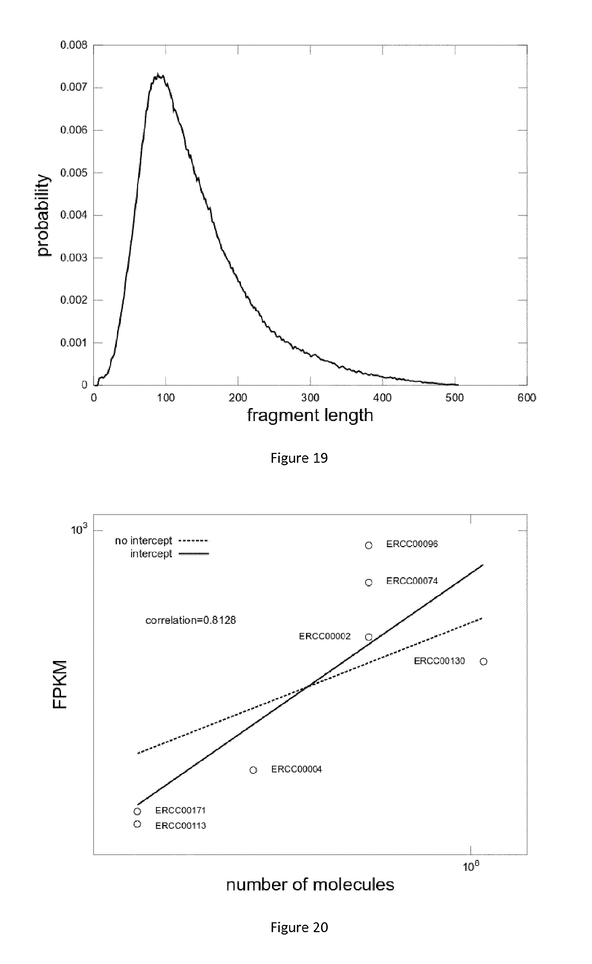

This section applies the methods developed in the previous sections for the estimation of $p(c=m|t=i,f=m)$ to the data of an NGS experiment. For this NGS experiment 500ng RNA input of Universal Human Reference RNA [7] were sequenced as part of a larger sample on an Illumina HiSeq 2000 chemistry version 3, producing 50 base pair long single end reads. The SENSE kit revision date 11.02.2013 [6] was used to prepare the library. SENSE is a stranded library preparation protocol and the start of a fragment can therefore be easily determined for each read. The length distribution of the resulting fragments is depicted in Figure 19 which was derived from the bioanalyser trace. The x-axis of Figure 19 gives the length of the fragments in base pairs and Figure 19 therefore shows that the peak of the fragment length distribution is located at around 100 base pairs. The fragment length distribution has a steep incline starting at zero and a heavy tail ending at around 500 base pairs and hence resembles a Gamma distribution. In order to measure the correlation between calculated and true concentration values 50ng ERCC reference RNA [3] with known concentration was spiked into the sample. The ERCC reference RNA consists of 96 synthetic transcripts which have minimal overlap with most common genomes. As a result, reads emanating from the ERCC transcripts most often map uniquely to the ERCC transcripts. The seven ERCC genes used in the following analysis where chosen due to the relatively large number of reads in the experiment that mapped to their annotation. This allows to obtain good estimates of the distribution of their fragment start sites. The identifiers of the seven ERCC genes used in this study are given in the first column of Table 3, whereas the number of their molecules and their length are given in the second and third column of Table 3. Cufflinks uses an adjusted length value to calculate the FPKM of a transcript, which is given in the fourth column of Table 3. According to [9] the adjusted length $\tilde{l}(t)$ of a transcript $t$ is given by

$$\tilde{l}(t) = \sum_{i=1}^{l(t)} F(i)\big(l(t) - i + 1\big)$$

$$(47)$$

where $F(i)$ is the probability of observing a fragment of length $i$, which is given by the distribution in 18. The number of reads mapping to the annotation of the ERCC transcripts is given in the fifth column. Substituting this number into the definition of the FPKM value (1) together with the transcript length, adjusted transcript length and total number of mapping reads in the experiment, which is 3,215,638, results in the values in the last two columns of Table 3.

Table 3: ERCC statistics, FPKM and adjusted length FPKM (AL-FPKM) values. The NGS experiment contained a total of 3,215,638 mapping reads.

| transcript | nr. molecules | length | adjusted length | reads | FPKM | AL-FPKM |
|---|---|---|---|---|---|---|
| ERCC00002 | 5.42E+07 | 1061 | 919.88 | 1934 | 566.86 | 653.83 |
| ERCC00004 | 2.71E+07 | 523 | 381.88 | 469 | 278.87 | 381.93 |
| ERCC00074 | 5.42E+07 | 522 | 380.88 | 1272 | 757.79 | 1038.56 |
| ERCC00096 | 5.42E+07 | 1107 | 965.88 | 3288 | 923.67 | 1058.63 |
| ERCC00113 | 1.35E+07 | 840 | 698.88 | 565 | 209.17 | 251.41 |
| ERCC00130 | 1.08E+08 | 1059 | 917.88 | 1694 | 497.45 | 573.93 |
| ERCC00171 | 1.35E+07 | 505 | 363.92 | 363 | 223.54 | 310.19 |

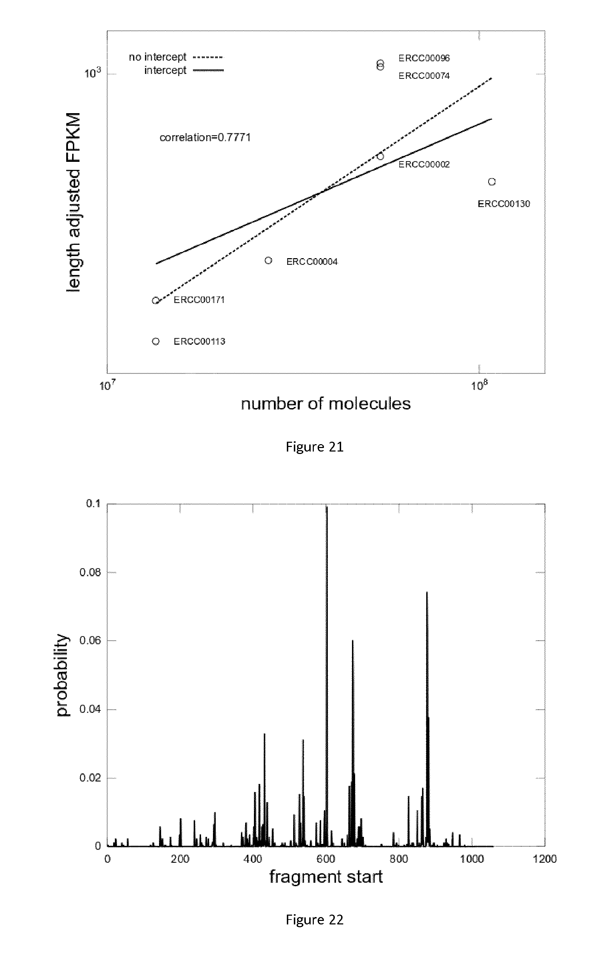

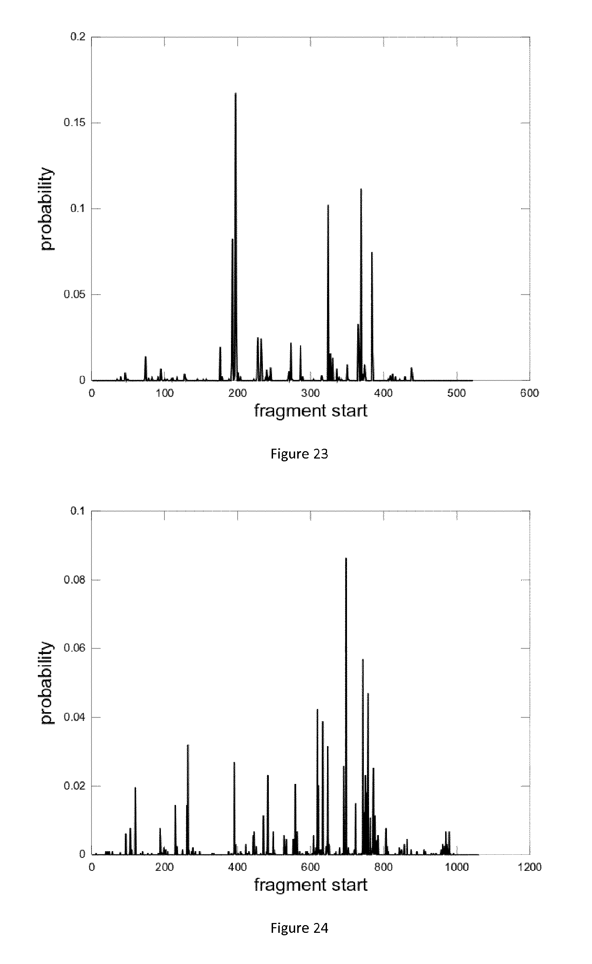

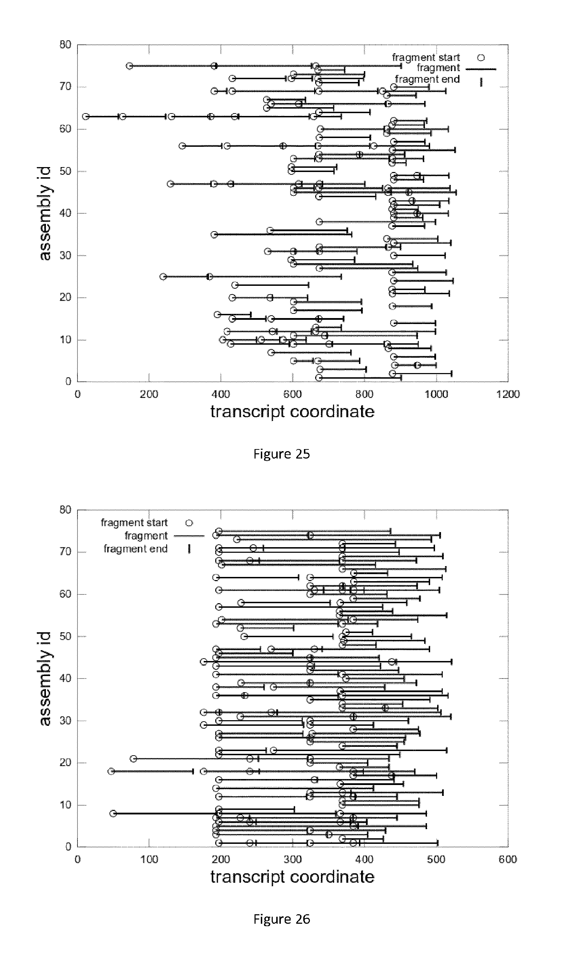

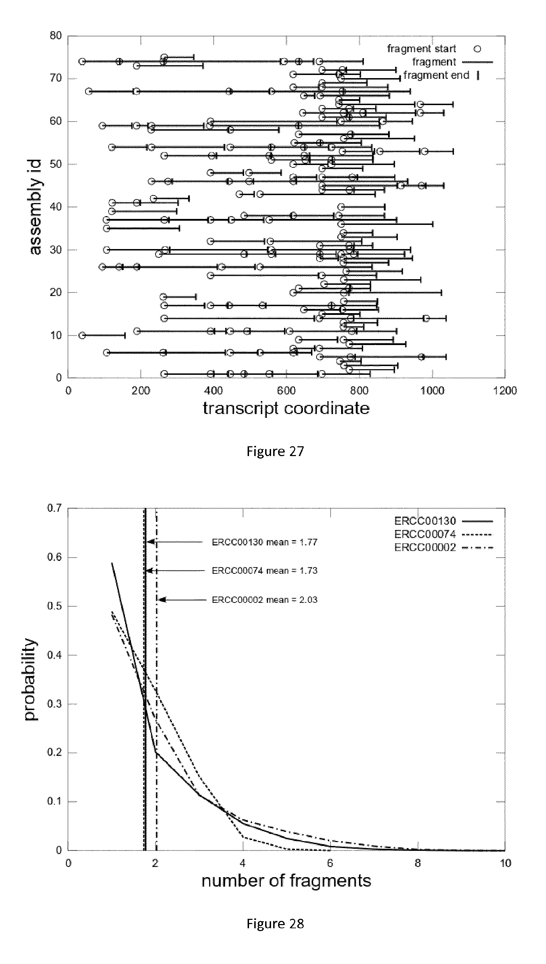

Figures 20 and 21 show the FPKM values for the length and adjusted length of the seven ERCC's plotted against their number of molecules. In addition, Figures 20 and 21 show the correlation between the number of molecules and the FPKM values together with the linear regression with and without intercept. Comparing Figure 20 and 21 shows that the correlation between the number of molecules and the FPKM value is 0.8128 which decreases to 0.7771 for the length adjusted FPKM. This decrease in correlation is mainly due to the overestimation of short transcripts which results in adjusted length FPKM values which are too high for short transcripts. In particular the adjusted length FPKM of ERCC00074 increases considerably in comparison to the standard FPKM value suggesting that the concentration of ERCC00074 in comparison to ERCC00130 is considerably higher, whereas the opposite is the case. Similarly the adjusted length FPKM value of the shorter transcript ERCC00171 is increased in comparison to the longer transcript ERCC00113, which results in an increased difference between the adjusted length FPKM values of these two ERCC transcripts even though they have the same number of molecules. The effect of overestimation of short transcripts by the FPKM and adjusted length FPKM value is well documented in the literature [4] but has not reduced their popularity as a normalization method. Figures 22, 23 and 24 show the distributions of the fragment start sites for transcripts ERCC00130, ERCC00074 and ERCC00002, respectively. These figures show that the distributions are very spiky and concentrated around a relatively small number of peaks. The location and height of these peaks differs significantly between different ERCC transcripts and reflects the preference of the SENSE sequencing primers to bind to certain sequences within the ERCC transcripts. In comparison to Figures 22 and 23 the start site distribution of the fragments of ERCC00002 in Figure 24 appears to be more evenly distributed along the full length of the transcript. The start site distribution of the fragments of ERCC00130 in Figure 22, on the other hand, is concentrated in the region between 400 and 900 base pairs. As a result, an assembly of ERCC00130 generated by the FSS will on average consist of fewer fragments than an assembly of ERCC00002. This, again, implies that for the same number of fragments the FSS will generate fewer copies of ERCC00002 than of ERCC00130 and thus employing the estimate of $p(f=m|t=i,c=n)$ derived by the FSS to estimate of $p(c=n|t=i,f=m)$ should have the desired effect of reducing the overestimated concentration of ERCC00002 in comparison to ERCC00130 in Figures 20 and 21. This effect is also visualized by Figures 25 and 27 which show 75 randomly drawn assemblies generated from 100,000 fragments with length distribution according to Figure 19 and start site distribution according to Figures 22 and 24, respectively. As in the experiments in section 2.3 the FSS uses assemblies of fragments with a bounded distance of 15 base pairs. Under this constraint the FSS generated 56272 assemblies for ERCC00130 and 49332 assemblies for ERCC00002. In comparison to Figure 27, Figure 25 shows much shorter assemblies. This is also reflected by the smaller number of fragments that appear in Figure 25, which is 125 in comparison to 178 in Figure 27. The assemblies generated by the FSS therefore suggest that a single copy of transcript ERCC00130 generates fewer fragments than a single copy of transcript ERCC00002. Comparing Figure 25 and 27, on the other hand, shows that transcript ERCC00074 has fewer short and fewer long assemblies than transcript ERCC00130 but a larger number of assemblies in the middle range, which results in 130 fragments in Figure 26 similar to the 125 fragments in Figure 27. Thus a single copy of transcript ERCC00130 produces on average the same number of fragments as a single copy of transcript ERCC00074 and for the same number of fragments their concentration values should therefore be roughly the same. This is in stark contrast to the FPKM values and adjusted length FPKM values in Table 3 whose normalization differs by more than a factor of 2 between ERCC00074 and ERCC00130, since the latter is about twice as long. Figure 28 shows the distributions of the number of fragments within an assembly generated by the FSS for transcripts ERCC00002, ERCC00074 and ERCC00130, which agree largely with the interpretation of Figures 25, 26 and 27. Table 4 gives the mean and variance of the distribution of the number of fragments within an assembly generated by the FSS for each of the seven ERCC transcripts. Substituting these values into (44) and (45) and calculating the probability of the number of copies of a transcript from (46), yields the expected number of transcripts in the final column of Table 4. As observed earlier, this value is largely similar to the number of reads divided by the mean number of fragments generated by a single copy of a transcript. The latter can therefore be interpreted as the factor by which the number of reads is normalized when estimating $p(c=n|t=i,f=m)$ from the $p(f=m|t=i,c=1)$ estimated by the FSS. Table 4 therefore shows that the number of reads of ERCC00074 and ERCC00130 are normalized to the same extend by the FSS in comparison to the FPKM value where the roughly twice as long ERCC00130 is normalized by a factor half as large as that for ERCC00074. Similarly ERCC00002 is normalized by a smaller factor than ERCC00130 in the FSS in comparison to the FPKM value where both are normalized with roughly the same factor due to their almost identical length. In summary this leads to a reduction of the normalized values of ERCC00002 and ERCC00074 in comparison to ERCC00130 when estimating $p(c=n|t=i,f=m)$ from the $p(f=m|t=i,c=1)$ generated by the FSS.

Table 4: Mean and variance of fragment number distributions $p(f = m \mid t = i, c = 1)$ of ERCC's and estimated number of transcripts.

| transcript | reads | mean | variance | expected number of transcripts |
|---|---|---|---|---|
| ERCC00002 | 1934 | 2.0270 | 1.9091 | 955 |
| ERCC00004 | 469 | 1.7360 | 0.7290 | 270 |
| ERCC00074 | 1272 | 1.7310 | 0.7194 | 738 |
| ERCC00096 | 3288 | 2.0172 | 1.6729 | 1637 |
| ERCC00113 | 565 | 2.5180 | 2.1469 | 225 |
| ERCC00130 | 1694 | 1.7738 | 1.3855 | 952 |
| ERCC00171 | 363 | 1.9003 | 0.9727 | 191 |

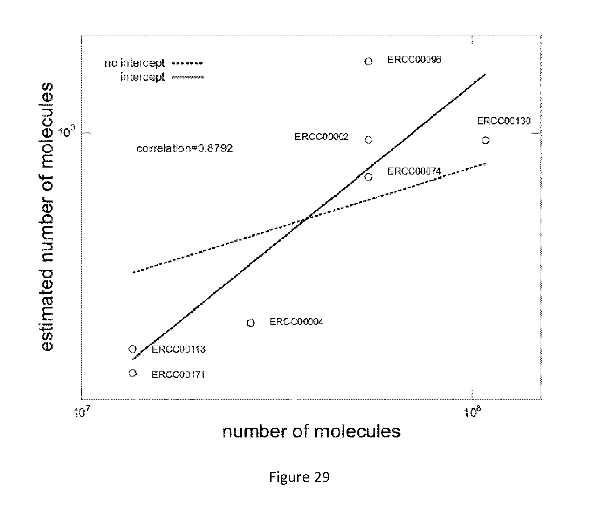

The estimated number of molecules is plotted against the true number of molecules in Figure 29 which shows an improvement in correlation between these two numbers in comparison to the FPKM value and length adjusted FPKM value in Figure 20 and 21. In particular the correlation of the estimated number of copies increases to 0.8792 from a correlation of 0.8128 for the FPKM value and 0.7771 for the length adjusted FPKM value. Furthermore, fitting a linear regression model with intercept results in a straight line with slope 0.9462 and an intercept of -4.4253. The fact that the slope of the linear regression is close to one indicates that the method described here correctly estimates the number of copies of transcripts and the intercept indicates that the library studied in this example does not contain transcripts with fewer than $10^{4.4253} = 26,626$ copies. This high cut-off is not surprising since the sequencing depth in this NGS experiment of 3,215,638 reads is relatively small.

## Conclusions

This document developed the fragment assembly algorithm (FAA) and its two special cases the fragment sequence synthesis (FSS) and fragment sequence reconstruction (FSR). With additional information on the connectivity of fragments the FSR can be used to reconstruct the original fragment sequences generated by a fragmentation process. As a result the FSR allows a direct estimate of the number of copies of a transcript by counting the reconstructed fragment sequences belonging to the transcript. The FSS, on the other hand, can be used to estimate $p(f=m|t=i,c=1)$ the probability distribution of the number of fragments generated by a single copy of transcript $t = i$. It was shown that for assemblies of fragments with bounded distance the FSS leads to rapidly converging estimates of $p(f = m \mid t = i, c = 1)$ which can be used as approximations of $p(f = m \mid t = i, c = 1)$ for assemblies with contiguous fragments. From estimates of $p(f=m|t=i,c=1)$

estimates of $p(f=m|t=i,c=n)$ can be derived either via decomposing $m$ into all possible sums consisting of $n$ summands or by application of the central limit theorem. In experiments on ERCC spike-in's of an NGS data set it was shown that application of the central limit theorem in conjunction with the FSS yields estimates of $p(c=n|t=i,f=m)$ which have a higher correlation with the true number of molecules of a transcript than both the FPKM and length adjusted FPKM values. In addition, fitting a linear regression with intercept to the true and estimated number of molecules yields a slope close to 1, which implies that the method described here produces in fact a proper estimate of the number of molecules of a transcript.

## References

[1] Simon Anders and Wolfgang Huber. Differential expression analysis for sequence count data. Genome Biol, 11(10):R106, 2010.

[2] James H Bullard, Elizabeth Purdom, Kasper D Hansen, and Sandrine Dudoit. Evaluation of statistical methods for normalization and differential expression in mrna-seq experiments. BMC Bioinformatics, 11:94, 2010.

[3] Lichun Jiang, Felix Schlesinger, Carrie A. Davis, Yu Zhang, Renhua Li, Marc Salit, Thomas R. Gingeras, and Brian Oliver. Synthetic spike-in standards for rna-seq experiments. Genome Research, 21(9):1543-1551, 2011.

[4] Bo Li, Victor Ruotti, Ron M. Stewart, James A. Thomson, and Colin N. Dewey. Rna-seq gene expression estimation with read mapping uncertainty. Bioinformatics, 26(4):493-500, 2010.

[5] Ali Mortazavi, Brian A Williams, Kenneth McCue, Lorian Schaeffer, and Barbara Wold. Mapping and quantifying mammalian transcriptomes by rna-seq. Nat Methods, 5(7):621-628, Jul 2008.

[6] Courtney Nadeau and Alexander Seitz. Sense: a fast rna-seq library preparation protocol with superior strand specificity. Nat Methods, December 2012.

[7] Natalia Novoradovskaya, Michael Whitfield, Lee Basehore, Alexey Novoradovsky, Robert Pesich, Jerry Usary, Mehmet Karaca, Winston Wong, Olga Aprelikova, Michael Fero, Charles Perou, David Botstein, and Jeff Braman. Universal reference rna as a standard for microarray experiments. BMC Genomics, 5(1):20, 2004.

[8] Mark D Robinson and Alicia Oshlack. A scaling normalization method for differential expression analysis of rna-seq data. Genome Biol, 11(3):R25, 2010.

[9] Cole Trapnell, Brian A Williams, Geo Pertea, Ali Mortazavi, Gordon Kwan, Marijke J van Baren, Steven L Salzberg, Barbara J Wold, and Lior Pachter. Transcript assembly and quantification by RNA-Seq reveals unannotated transcripts and isoform switching during cell differentiation. Nat Biotechnol, 28(5):511-515, May 2010.

## Claims

1. A method for determining an amount of nucleic acid molecule copies of a preselected nucleic acid molecule in a sample, comprising the steps of

   a) generating fragments of the preselected nucleic acid molecule,
   b) providing the amount of fragments for said preselected nucleic acid molecule,
   c) providing a distribution of fragment coverage over a genetic coordinate for said preselected nucleic acid molecule,
   d1) generating a plurality of virtual fragments based on the genetic coordinates of the preselected nucleic acid molecule, so that the generated virtual fragments have substantially the distribution of fragment coverage over the genetic coordinate according to step c), or d2) determining the nucleic acid sequence of fragments obtained in step a) and providing each nucleic acid sequence of a fragment as virtual fragment;
   thereby obtaining a plurality of virtual fragments,
   e) assembling the obtained virtual fragments from step d) into fragment sequences, wherein each virtual fragment is assembled into a fragment sequence which is composed of matching virtual fragments, until all virtual fragments of step d) have been either assembled into a fragment sequence or do not match with any other non-assembled virtual fragment or fragment sequence and all fragment sequences do not match with any other fragment sequence, wherein each final fragment sequence and non-assembled virtual fragment is regarded as a virtual copy,

f) obtaining the amount of virtual fragments from step d) per virtual copy obtained in step e),

g) whereby the amount of the copies of a preselected nucleic acid molecule in a sample can be calculated from the fragment amount of step b) and the amount of annealed fragments per virtual copy of step f).

2. The method of claim 1, wherein the preselected nucleic acid molecule is selected from a DNA or RNA molecule in a sample, preferably a transcript, especially an mRNA.

3. The method of claim 1 or 2, wherein the fragments of step a) and/or (selected independently or dependently) the virtual fragments of step d) have an average size of 40 to 2000 nucleotides in length, preferably 60 to 1000 nucleotides in length, especially preferred 80 to 800 nucleotides in length.

4. The method of any one of claims 1 to 3, wherein fragmentation is random fragmentation, preferably by cutting by physical means, in particular preferred by sonication, shearing or elevated temperature.

5. The method of any one of claims 1 to 4, wherein the distribution of fragment coverage over a genetic coordinate is a distribution on the fragment start and fragment end for each fragment, a fragment start site distribution or a distribution of at least one nucleotide of each fragment at a preselected position from a fragment 3' or 5' terminus.

6. The method of any one of claims 1 to 5, wherein the distribution of fragment coverage over a genetic coordinate is determined by a method comprising assigning fragment sequencing data to genetic coordinates of said preselected nucleic acid molecule thereby obtaining a data set of fragment genetic coordinate coverage.

7. The method of any one of claims 1 to 5, wherein the distribution of fragment coverage over a genetic coordinate is determined by using a reference nucleic acid, which is fragmented, and the coverage of fragments over a genetic coordinate of said reference nucleic acid is determined, thereby obtaining a reference coverage, which reference coverage is fitted in length to the genetic coordinate of the preselected nucleic acid molecule.

8. The method of any one of claims 1 to 7, wherein step d1) is repeated at least once, thereby producing a different set of generated virtual fragments and/or virtual fragments are continuously generated according to step d1) and assembled according to step e), preferably until the amount of virtual fragments of step f) converges.

9. The method of claim 8, wherein at least 20, preferably at least 50, even more preferred at least 200, random sets of generated virtual fragments are generated and/or at least 10, preferably at least 20, at least 30, at least 40, at least 50, at least 60, at least 60, at least 80, at least 100, at least 125, at least 150, at least 200, at least 300, at least 400, or at least 500, virtual fragments per nucleotide in length of the preselected nucleic acid molecule are generated.

10. The method of any one of claims 1 to 7, wherein step d2) 1% to 100 %, preferably 2% to 80%, even more preferred 3% to 40% of the fragments are sequenced.

11. The method of any one of claims 1 to 10, wherein in the assembly of two matching virtual fragments within one fragment sequence that are adjacent in terms of their sequence in step e) the start in genome coordinates of a subsequent fragment follows immediately after the end in genome coordinates of a preceeding virtual fragment (exact match) or the start of a subsequent virtual fragment and the end of a preceeding virtual fragment are located within a window of +/- 1 to +/- 200 nucleotides (inexact match).

12. The method of any one of claims 1 to 11, wherein the assembly step e) is repeated with a different order of virtual fragments to be assembled, preferably wherein at least 10 different sets of assembled fragment sequences, at least 100, or especially at least 1000, different sets of assembled fragment sequences are generated.

13. The method of any one of claims 1 to 12, wherein the preselected nucleic acid molecule has 500 to 10000000 nucleotides in length, preferably 1000 to 1000000 nucleotides in length, even more preferred 5000 to 500000 nucleotides in length.

**14.** The method of any one of claims 1 to 13, further comprising the step of providing a distribution of fragment lengths of the fragments of step a) and wherein in step d1) the virtual fragments are generated so that the generated virtual fragments have substantially the distribution of fragment lengths.

**15.** A computer readable memory device comprising a computer program product for performing a method of any one of claims 1 to 14 on a computer.

Figure 1a

Figure 1b

Figure 2a

Figure 2b

fragment statistics

sampled fragments

sample p(r|t=i)

select sequence
to extend by $r_n$

fragment sequences assembled
from fragments $r_1,...,r_n$

$p(f=m|t=i,c=1)$

derive statistics
for large n

Figure 3

Figure 4

Figure 5

Figure 6

Figure 7

Figure 8

Figure 9

Figure 10

Figure 11

Figure 12

Figure 13

Figure 14

Figure 15

Figure 16

Figure 17

Figure 18

Figure 19

Figure 20

Figure 21

Figure 22

Figure 23

Figure 24

Figure 25

Figure 26

Figure 27

Figure 28

Figure 29

Europäisches
Patentamt

European
Patent Office

Office européen
des brevets

# EUROPEAN SEARCH REPORT

Application Number

EP 13 19 1170

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2009/091798 A1 (HELICOS BIOSCIENCES CORP [US]; LIPSON DORON [US]; RAZ TAL [US]) 23 July 2009 (2009-07-23) * example 7 * | 1-15 | INV. C12Q1/68 G06F19/22 |
| A,D | ROBINSON MARK D ET AL: "A scaling normalization method for differential expression analysis of RNA-seq data", GENOME BIOLOGY, BIOMED CENTRAL LTD., LONDON, GB, vol. 11, no. 3, 2 March 2010 (2010-03-02), page R25, XP021070870, ISSN: 1465-6906 * the whole document * | 1-15 | |
| A | LI JUN ET AL: "Modeling non-uniformity in short-read rates in RNA-Seq data", GENOME BIOLOGY, BIOMED CENTRAL LTD., LONDON, GB, vol. 11, no. 5, 11 May 2010 (2010-05-11), page R50, XP021085605, ISSN: 1465-6906, DOI: 10.1186/GB-2010-11-5-R50 * the whole document * | 1-15 | TECHNICAL FIELDS SEARCHED (IPC) C12Q |
| A | BO LI ET AL: "RSEM: accurate transcript quantification from RNA-Seq data with or without a reference genome", BMC BIOINFORMATICS, BIOMED CENTRAL, LONDON, GB, vol. 12, no. 1, 4 August 2011 (2011-08-04) , page 323, XP021104619, ISSN: 1471-2105, DOI: 10.1186/1471-2105-12-323 * the whole document * | 1-15 | |

-/--

The present search report has been drawn up for all claims

| Place of search | Date of completion of the search | Examiner |
|---|---|---|
| The Hague | 3 February 2014 | Cornelis, Karen |

EPO FORM 1503 03.82 (P04C01)

Europäisches
Patentamt

European
Patent Office

Office européen
des brevets

# EUROPEAN SEARCH REPORT

Application Number

EP 13 19 1170

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | ALI MORTAZAVI ET AL: "Mapping and quantifying mammalian transcriptomes by RNA-Seq", NATURE METHODS, NATURE PUBLISHING GROUP, GB, vol. 5, no. 7, 1 July 2008 (2008-07-01), pages 621-628, XP008148418, ISSN: 1548-7091, DOI: 10.1038/NMETH.1226 * the whole document * | 1-15 | |

TECHNICAL FIELDS
SEARCHED    (IPC)

The present search report has been drawn up for all claims

| Place of search | Date of completion of the search | Examiner |
|---|---|---|
| The Hague | 3 February 2014 | Cornelis, Karen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 13 19 1170

This annex lists the patent family members relating to the patent documents cited in the above-mentioned European search report.
The members are as contained in the European Patent Office EDP file on
The European Patent Office is in no way liable for these particulars which are merely given for the purpose of information.

03-02-2014

| Patent document cited in search report | Publication date | Patent family member(s) | Publication date |
|---|---|---|---|
| WO 2009091798 A1 | 23-07-2009 | NONE | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2013110410 A1
- WO 2009085412 A1
- WO 2009091798 A1
- EP 13175774 A
- WO 2011070155 A

### Non-patent literature cited in the description

- **SIMON ANDERS ; WOLFGANG HUBER.** Differential expression analysis for sequence count data. *Genome Biol,* 2010, vol. 11 (10), R106
- **JAMES H BULLARD ; ELIZABETH PURDOM ; KASPER D HANSEN ; SANDRINE DUDOIT.** Evaluation of statistical methods for normalization and differential expression in mrna-seq experiments. *BMC Bioinformatics,* 2010, vol. 11, 94
- **LICHUN JIANG ; FELIX SCHLESINGER ; CARRIE A. DAVIS ; YU ZHANG ; RENHUA LI ; MARC SALIT ; THOMAS R. GINGERAS ; BRIAN OLIVER.** Synthetic spike-in standards for rna-seq experiments. *Genome Research,* 2011, vol. 21 (9), 1543-1551
- **BO LI ; VICTOR RUOTTI ; RON M. STEWART ; JAMES A. THOMSON ; COLIN N. DEWEY.** Rna-seq gene expression estimation with read mapping uncertainty. *Bioinformatics,* 2010, vol. 26 (4), 493-500
- **ALI MORTAZAVI ; BRIAN A WILLIAMS ; KENNETH MCCUE ; LORIAN SCHAEFFER ; BARBARA WOLD.** Mapping and quantifying mammalian transcriptomes by rna-seq. *Nat Methods,* July 2008, vol. 5 (7), 621-628
- **COURTNEY NADEAU ; ALEXANDER SEITZ.** Sense: a fast rna-seq library preparation protocol with superior strand specificity. *Nat Methods,* December 2012
- **NATALIA NOVORADOVSKAYA ; MICHAEL WHITFIELD ; LEE BASEHORE ; ALEXEY NOVORADOVSKY ; ROBERT PESICH ; JERRY USARY ; MEHMET KARACA ; WINSTON WONG ; OLGA APRELIKOVA ; MICHAEL FERO.** Universal reference rna as a standard for microarray experiments. *BMC Genomics,* 2004, vol. 5 (1), 20
- **MARK D ROBINSON ; ALICIA OSHLACK.** A scaling normalization method for differential expression analysis of rna-seq data. *Genome Bio,* 2010, vol. 11 (3), R25
- **COLE TRAPNELL ; BRIAN A WILLIAMS ; GEO PERTEA ; ALI MORTAZAVI ; GORDON KWAN ; MARIJKE J VAN BAREN ; STEVEN L SALZBERG ; BARBARA J WOLD ; LIOR PACHTER.** Transcript assembly and quantification by RNA-Seq reveals unannotated transcripts and isoform switching during cell differentiation. *Nat Biotechnol,* May 2010, vol. 28 (5), 511-515